# FASCICULE DE BREVET EUROPEEN

(11) **EP 4 449 114 B1**
(45) Date de publication et mention de la délivrance du brevet: **15.07.2026**
(21) Numéro de dépôt: 22847559.6
(22) Date de dépôt: 15.12.2022
(51) Int. Cl.: G01N 31/22, C12Q 1/26, G01N 21/78, G01N 33/52, G01N 33/84

(54) **SYSTÈME DE DÉTECTION COLORIMÉTRIQUE POUR LA DÉTECTION DE COMPOSÉS CHIMIQUES AU MOYEN D'UN INDICATEUR COLORÉ DE LA FAMILLE DES HYDRAZONES**
KOLORIMETRISCHES DETEKTIONSSYSTEM ZUM NACHWEIS VON CHEMISCHEN VERBINDUNGEN MITTELS EINES FARBINDIKATORS AUS DER FAMILIE DER HYDRAZONE
COLORIMETRIC DETECTION SYSTEM FOR THE DETECTION OF CHEMICAL COMPOUNDS BY MEANS OF A COLOR INDICATOR FROM THE HYDRAZONE FAMILY

(30) Priorité: 16.12.2021 FR 2113669
(43) Date de publication de la demande: 23.10.2024
(73) Titulaire: Commissariat à l'Energie Atomique et aux Energies Alternatives, 75015 Paris (FR)
(72) Inventeur: TERMEAU, Lucile, 38054 Grenoble Cedex 09 (FR); PENLOU, Sébastien, 38054 Grenoble Cedex 09 (FR); CARELLA, Alexandre, 38054 Grenoble Cedex 09 (FR)
(74) Mandataire: Santarelli
(86) Numéro de dépôt international: PCT/FR2022/052383
(87) Numéro de publication internationale: WO 2023/111473

(56) Documents cités:
- US-A- 4 247 297
- US-A- 5 474 907
- US-A- 5 776 719
- US-A1- 2002 119 508

## Description

### DOMAINE TECHNIQUE

La présente invention a trait à un nouveau système de détection colorimétrique utilisable pour la détection de composés chimiques, en particulier, de composés toxiques de guerre, au moyen d'un indicateur coloré de la famille des hydrazones et à un procédé de détection de la présence ou de l'absence de composés chimiques mettant en œuvre ce système de détection colorimétrique.

En particulier, la présente invention peut trouver application pour la détection de composés chimiques toxiques, tels que les composés toxiques de guerre et, en particulier, les composés organophosphorés ; les composés toxiques industriels chimiques (connus sous la dénomination TIC), les pesticides.

De manière générale, les composés organophosphorés se présentent sous la forme de composés organiques présentant une toxicité avérée pour l'organisme humain. En effet, ces composés peuvent être impliqués dans le mécanisme d'inhibition des sérineprotéases et, en particulier, de l'acétylcholinestérase, qui intervient dans les jonctions synaptiques et dont le dérèglement de l'activité peut empêcher le relâchement musculaire et ainsi provoquer la mort par asphyxie.

Ces composés peuvent être compris dans la formulation d'insecticides, de pesticides ou encore d'agents chimiques de combat (tels que les composés organosphosphorés de la série G, comme le sarin (GB, CAS n°107-44-8) ou les composés organophosphorés de la série V, comme le VX (CAS n°50782-69-9) et du fait de la létalité élevée de ces composés, de leur prolifération, il s'avère important de pouvoir disposer de systèmes de détection et d'identification préliminaire

Certains systèmes de détection utilisés à ce jour sont basés sur des technologies impliquant des moyens de mesure physique, telles que la spectroscopie à mobilité ionique, la photométrie de flamme, les spectroscopies IR et Raman avec, pour difficultés, que ces systèmes nécessitent un appareillage onéreux complexe et pas forcément adaptés à tous les milieux d'intervention en termes de masse et d'encombrement, auxquelles s'ajoute l'expertise de l'opérateur à considérer.

Au vu de ce qui existe, les auteurs de la présente invention se sont orientés vers la conception d'un système de détection utilisable pour la détection de composés chimiques, tels que des composés organophosphorés, à partir d'un indicateur coloré spécifique.

### EXPOSÉ DE L'INVENTION

Ainsi, l'invention a trait à un système de détection colorimétrique utilisable pour la détection d'un composé chimique comprenant un support comprenant un indicateur coloré dudit composé chimique à détecter, caractérisé en ce que l'indicateur coloré est un composé hydrazone conjugué. Le système de détection de la présente invention est défini dans la revendication 1.

Par indicateur coloré (dit également indicateur de détection colorimétrique), il s'entend, classiquement, une substance chimique qui prend au moins une couleur caractéristique en présence d'un composé chimique soit, en d'autres termes, une substance chimique qui présente au moins deux états colorés, un état coloré existant lorsque la substance chimique n'est pas en présence du composé chimique à détecter et au moins un autre état coloré lorsque la substance chimique est en présence du composé chimique à détecter.

Le support constitutif du système de détection peut être, en particulier, un support comprenant des fibres de verre ou est un support en papier (par exemple, du papier de chromatographie), avec une préférence pour le support comprenant des fibres de verre, ou est un support comprenant une poudre (par exemple, une poudre de silice, une poudre de polyéthylène) étant entendu que ce support doit être apte à être imprégné le composé hydrazone conjugué.

Par hydrazone conjugué, il s'entend un composé comprenant une fonction hydrazone de formule -NH-N=C-, dont la double liaison est conjuguée à une autre double liaison, soit, en d'autres termes, que l'atome de carbone porteur de la double liaison de la fonction hydrazone est lié à un autre atome de carbone porteur d'une double liaison, ce qui peut être représenté schématiquement par la formule (I) suivante :

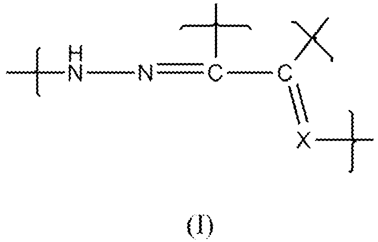

X indiquant un atome de carbone ou un hétéroatome, les accolades indiquant que les atomes sont liés à d'autres atomes pour atteindre leur valence, cette formule couvrant également les formes tautomères. Plus loin, lorsqu'il sera fait mention de l'atome d'azote de la fonction hydrazone, il s'entend de l'atome d'azote non porteur de la double liaison, cette atome d'azote étant celui représenté à gauche de la formule (I). Il est entendu, également, que lorsque X est un atome de carbone, il comportera une valence supplémentaire par rapport à ce qui est représenté sur la formule (I).

Plus spécifiquement, l'autre carbone porteur d'une double liaison peut appartenir à un groupe aromatique, éventuellement hétéroaromatique ou peut appartenir à un groupe éthylénique.

Selon un premier mode de réalisation, le composé hydrazone conjugué comprend au moins un groupe cyclique lié à l'atome de carbone porteur de la double liaison de la fonction hydrazone et comprend, en outre, un groupe aromatique porteur d'au moins un groupe électroattracteur (tel que NO₂), lié à l'atome d'azote de la fonction hydrazone.

Des composés hydrazones répondant aux spécificités de ce premier mode de réalisation peuvent être des composés dont le ou les groupes cycliques liés à l'atome de carbone porteur de la double liaison de la fonction hydrazone sont des groupes cycliques carbonés (c'est-à-dire dont les atomes du cycle sont tous des atomes de carbone, ce qui n'exclut pas que ces atomes de carbone puissent être liés à des groupes porteurs d'un ou plusieurs hétéroatomes), par exemple des groupes aromatiques carbonés et, encore plus spécifiquement, des composés hydrazones dont le ou les groupes cycliques liés à l'atome de carbone porteur de la double liaison de la fonction hydrazone sont des groupes cycliques carbonés (par exemple des groupes aromatiques carbonés) et le groupe aromatique porteur d'au moins un groupe électroattracteur est un groupe aromatique porteur d'au moins un groupe NO₂.

Des composés hydrazones répondant à ces critères pouvant répondre à l'une des formules (II), (III), (IV), (V), (VI) et (VI') suivantes : 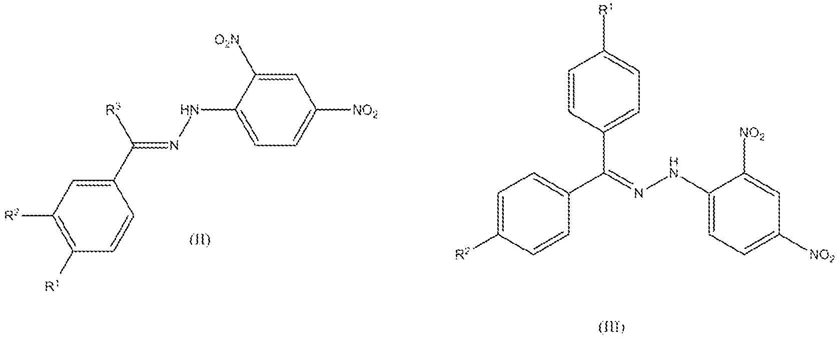 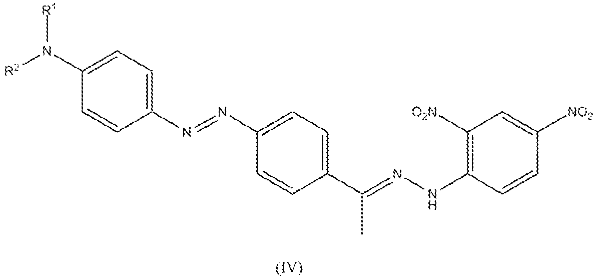 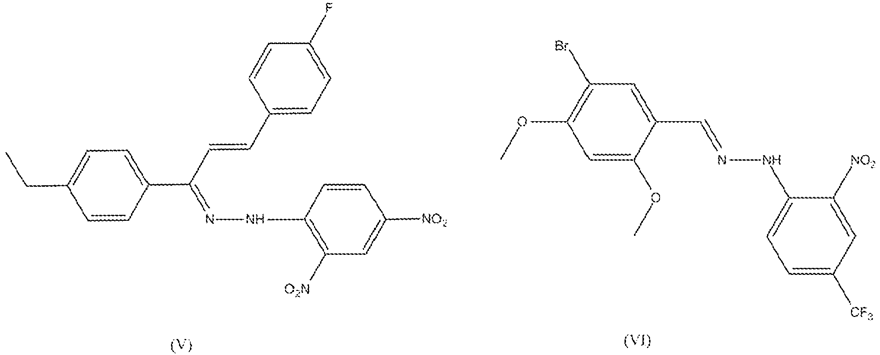 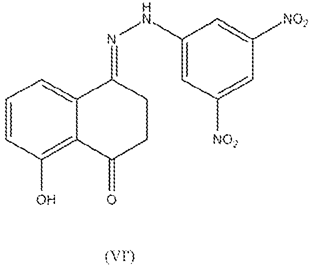 pour lesquelles :
- pour la formule (II), R¹ représente -N(CH₃)₂, -CH₃, -OCH₃, -OH, - SCH₃ ou -NO₂ ; R² représente -H ou -OCH₃; R³ représente -H ou -CH₃;
- pour la formule (III), R¹ et R² représentent, indépendamment l'un de l'autre, -H, -N(CH₃)₂, -OCH₃ ou -NO₂ ;
- pour la formule (IV), R¹ et R² représentent, indépendamment l'un de l'autre, -CH₃ ou -CH₂-CH₃.

Plus spécifiquement, des composés particuliers entrant dans la définition des composés de formule (II) sont les suivants :
- le composé pour lequel R¹ représente -N(CH₃)₂, R² représente - H et R³ représente H (dit composé ci-après « composé 1a ») ;
- le composé pour lequel R¹ représente -CH₃, R² représente H et R³ représente H (dit composé ci-après « composé 1b ») ;
- le composé pour lequel R¹ représente -OCH₃, R² représente -H et R³ représente H (dit composé ci-après « composé 1c ») ;
- le composé pour lequel R¹ représente -OH, R² représente - OCH₃ et R³ représente H (dit composé ci-après « composé 1d ») ;
- le composé pour lequel R¹ représente -SCH₃, R² représente -H et R³ représente H (dit composé ci-après « composé 1e ») ; et
- le composé pour lequel R¹ représente -NO₂, R² représente -H et R³ représente -CH₃ (dit composé ci-après « composé 1f »).

Plus spécifiquement, des composés particuliers entrant dans la définition des composés de formule (III) sont les suivants :
- le composé pour lequel R¹ représente -H et R² représente -H (dit composé ci-après « composé 3a ») ;
- le composé pour lequel R¹ représente -N(CH₃)₂, R² représente - N(CH₃)₂ (dit composé ci-après « composé 3b ») ; et
- le composé pour lequel R¹ représente -OCH₃ et R² représente - OCH₃ (dit composé ci-après « composé 3c »).

Plus spécifiquement, des composés particuliers entrant dans la définition des composés de formule (IV) sont les suivants :
- le composé pour lequel R¹ représente -CH₃ et R² représente - CH₃ (dit composé ci-après « composé 5a ») ; et
- le composé pour lequel R¹ représente -CH₂-CH₃ , R² représente -CH₂-CH₃ (dit composé ci-après « composé 5b »).

Des composés hydrazones répondant aux spécificités du premier mode de réalisation peuvent être également des composés dont le ou les groupes cycliques liés à l'atome de carbone porteur de la double liaison de la fonction hydrazone sont des groupes hétéroaromatiques (c'est-à-dire un groupe dont au moins un atome du ou des cycles est un hétéroatome, tel que O, N, S) et, plus spécifiquement, des composés hydrazones dont le ou les groupes cycliques liés à l'atome de carbone porteur de la double liaison de la fonction hydrazone sont des groupes hétéroaromatiques et le groupe aromatique porteur d'au moins un groupe électroattracteur est un groupe aromatique porteur d'au moins un groupe NO₂.

Des composés hydrazones répondant à ces critères peuvent répondre à l'une des formules (VII), (VIII), (IX), (X) et (XI) suivantes : 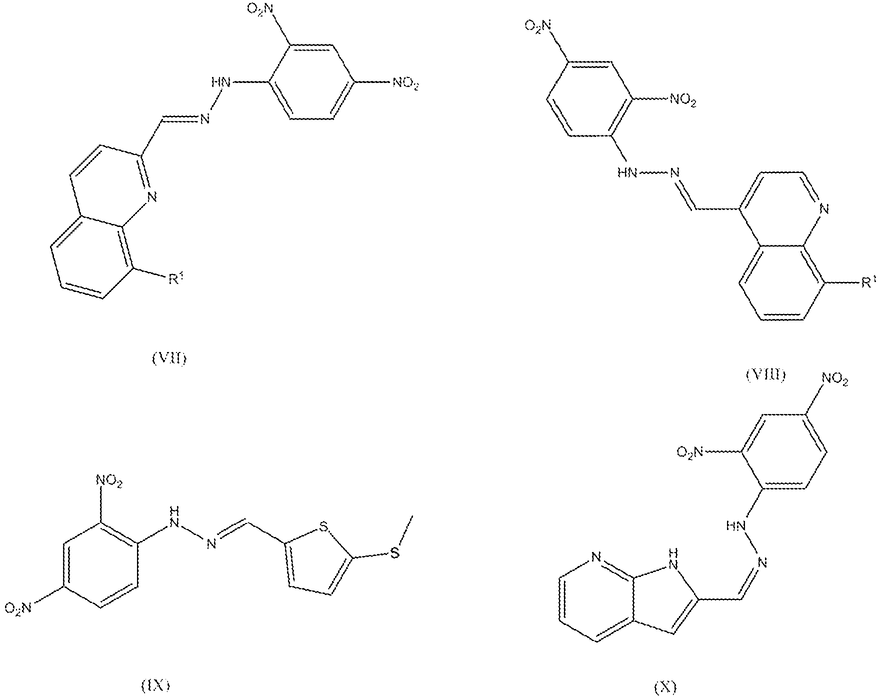 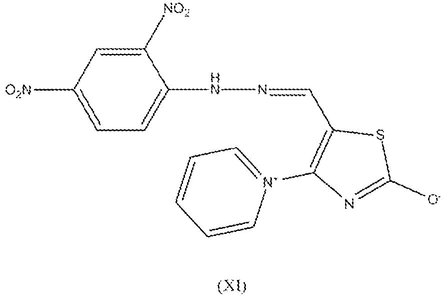 pour lesquelles :
- pour la formule (VII), R¹ représente -H ou -OH;
- pour la formule (VIII), R¹ représente -H ou -OH.

Plus spécifiquement, des composés particuliers entrant dans la définition des composés de formule (VII) ou (VIII) sont les suivants :
- le composé pour lequel R¹ représente -H (dit composé ci-après « composé 2a ») ;
- le composé pour lequel R¹ représente -OH (dit composé ci-après « composé 2b »).

Selon un deuxième mode de réalisation, le composé hydrazone conjugué comprend au moins un groupe éthylénique lié à l'atome de carbone porteur de la double liaison de la fonction hydrazone et comprenant, en outre, un groupe aromatique porteur d'au moins un groupe électroattracteur (tel que NO₂ ou un groupe hétéroaromatique) lié à l'atome d'azote de la fonction hydrazone, des composés particuliers répondant à cette spécificité répondant à l'une des formules (XII) ou (XIII) suivantes :

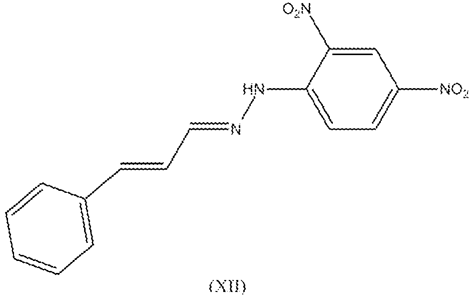

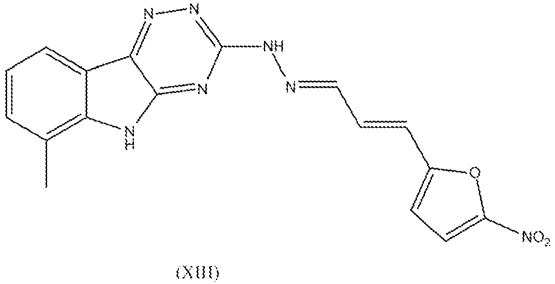

Selon un troisième mode de réalisation, le composé hydrazone conjugué comprend au moins un groupe aromatique porteur d'un groupe -OH lié à l'atome d'azote de la fonction hydrazone ou à l'atome de carbone porteur de la double liaison de la fonction hydrazone, des composés particuliers répondant à cette spécificité répondant à l'une des formules (XIV), (XV), (XVI), (XVII), (XVIII), (XIX) et (XX) suivante :

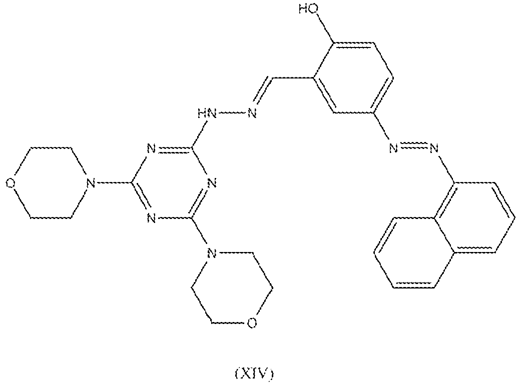

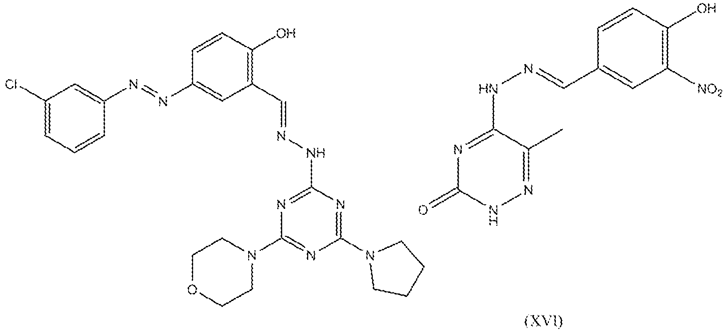

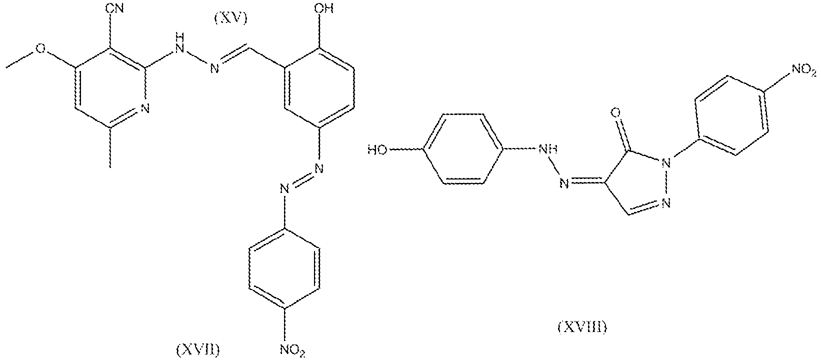

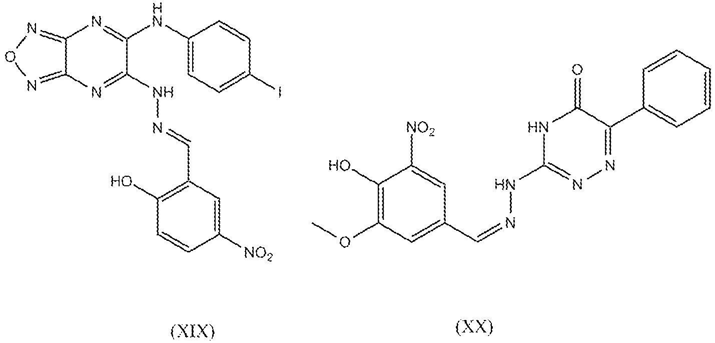

Selon un quatrième mode de réalisation, le composé hydrazone conjugué comprend un groupe hétéroaromatique lié, éventuellement via un groupe espaceur (tel qu'un groupe -CO-), à l'atome d'azote de la fonction hydrazone et un autre groupe hétéroaroaromatique lié à l'atome de carbone porteur de la double liaison de la fonction hydrazone ou comprend un groupe diazoaromatique lié à l'azote d'azote de la fonction hydrazone et un groupe hétéroaromatique englobant l'atome de carbone porteur de la double liaison de la fonction hydrazone, des composés particuliers répondant à cette spécificité répondant à l'une des formules (XXI), (XXII), (XXIII) et (XXIV) suivante :

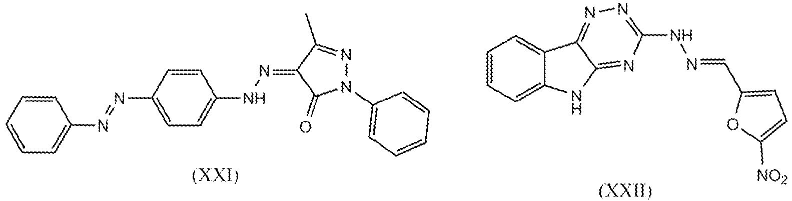

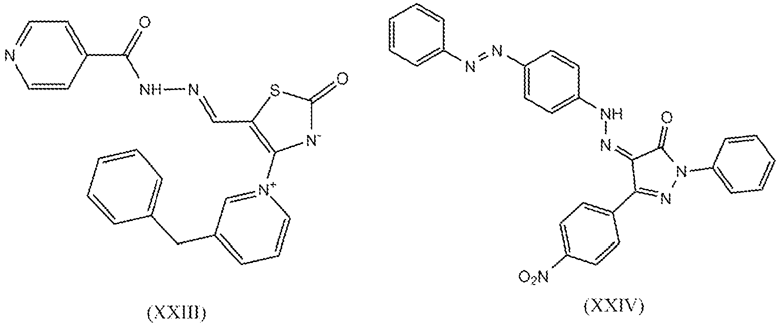

Parmi les composés hydrazones susmentionnés, certains sont nouveaux et sont l'objet de l'invention, ces composés répondant à l'une des formules (5a), (5b) et (X) suivantes :

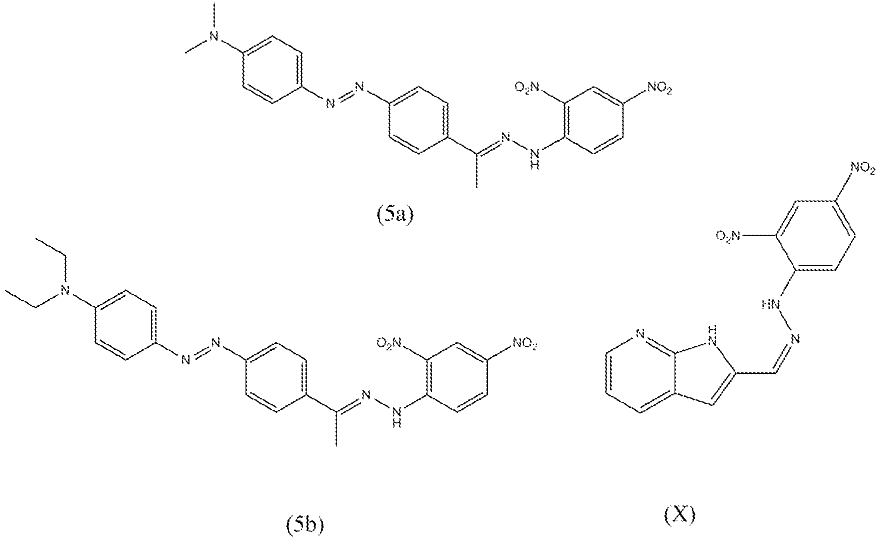

Ces composés peuvent être obtenus classiquement par une réaction de condensation entre un composé carbonyle (par exemple, une cétone ou un aldéhyde) et un composé comprenant une fonction hydrazine.

Les systèmes de détection conformes à l'invention peuvent être préparés par un procédé comprenant une étape de dépôt de l'indicateur indicateur coloré sur le support en projetant une encre comprenant l'indicateur coloré sur celui-ci.

Concernant les techniques de dépôt envisageables, il peut s'agir d'un dépôt à la micropipette, d'une impression (par exemple, *via* une imprimante Dimatix), d'un dépôt *via* des particules imprégnées par l'indicateur coloré approprié ou d'un dépôt par sérigraphie.

Enfin, l'invention a trait également à un procédé de détection de la présence ou de l'absence d'un composé chimique comprenant les étapes suivantes :
- une étape de mise en contact du ou des milieux, dont on veut détecter la présence ou l'absence dudit composé chimique avec le système de détection tel que défini ci-dessus ;
- une étape de déduction, en fonction du ou des éventuels virages chromatiques observés, de la présence ou de l'absence dudit composé chimique.

Cette étape de mise en contact peut consister à déposer en surface du système de détection une ou plusieurs gouttes distinctes de chaque milieu dont on veut analyser l'absence ou la présence d'un composé chimique.

Il s'entend que le système de détection colorimétrique devant être utilisé dans le cadre du procédé susmentionné doit être apte à détecter le composé chimique, dont on veut déterminer l'absence ou la présence.

Entre l'étape de mise en contact et l'étape de déduction, il peut être prévu un temps d'attente pour que, le cas échéant, le virage chromatique puisse avoir lieu.

Concernant l'étape de déduction, l'opérateur pourra se baser sur une échelle colorimétrique associée au système de détection, qui définira, pour tous les composés chimiques susceptibles d'être détectés par le système, le virage chromatique correspondant, cette échelle colorimétrique pouvant être déterminée, par des essais préalables, pour chacun des systèmes et les composés chimiques destinés à être détectés par lesdits systèmes. La déduction pourra se faire à l'œil nu ou, si nécessaire, *via* des moyens opto-électroniques.

Les composés susceptibles d'être détectés par le procédé de l'invention peuvent être des composés toxiques de guerre ; des composés toxiques industriels, des pesticides et plus spécifiquement, peuvent être des composés organophosphorés et, encore plus spécifiquement, des composés organophosphorés toxiques répondant à l'une des formules (XXV) et (XXVI) suivantes : 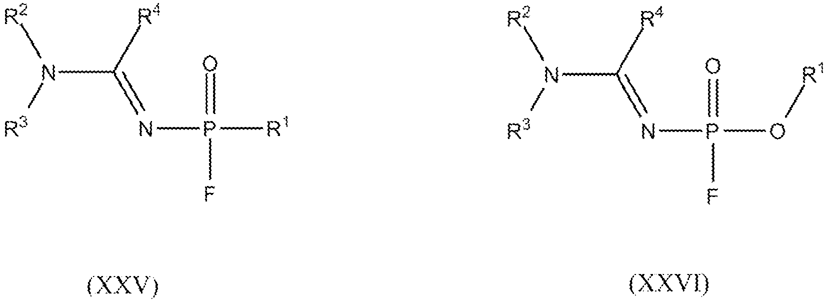 dans lesquelles :
- R¹ représente H ou un groupe alkyle, éventuellement cyclique, pouvant comprendre jusqu'à 10 atomes de carbone ;
- R² et R³ représentent, indépendamment l'un de l'autre, un groupe alkyle, éventuellement cyclique pouvant comprendre jusqu'à 10 atomes de carbone ;
- R⁴ représente H, un groupe alkyle, éventuellement cyclique, pouvant comprendre jusqu'à 10 atomes de carbone ou un groupe amino.

En particulier, les systèmes de détection, notamment lorsque le support est en fibres de verre et l'indicateur coloré est un composé hydrazone 1b, 1d, 1f, 2a, 2b ou 3c tels que définis ci-dessus ou un composé de formule (IX) telle que défini ci-dessus, sont particulièrement adaptés à la détection sélective de composés organophosphorés toxiques répondant à l'une des formules (XXV) et (XXVI) suivantes : 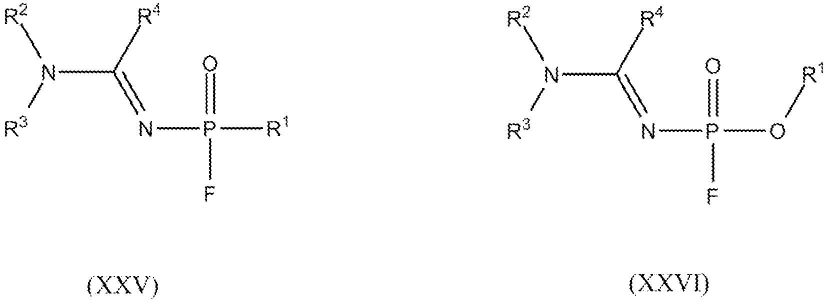 dans lesquelles :
- R¹ représente H ou un groupe alkyle, éventuellement cyclique, pouvant comprendre jusqu'à 10 atomes de carbone ;
- R² et R³ représentent, indépendamment l'un de l'autre, un groupe alkyle, éventuellement cyclique pouvant comprendre jusqu'à 10 atomes de carbone ;
- R⁴ représente H, un groupe alkyle, éventuellement cyclique, pouvant comprendre jusqu'à 10 atomes de carbone ou un groupe amino.

Des composés organophosphorés spécifiques entrant dans cette catégorie sont les composés spécifiques répondant à l'une des formules (XXVII), (XXVIII), (XXIX) et (XXX) suivantes :

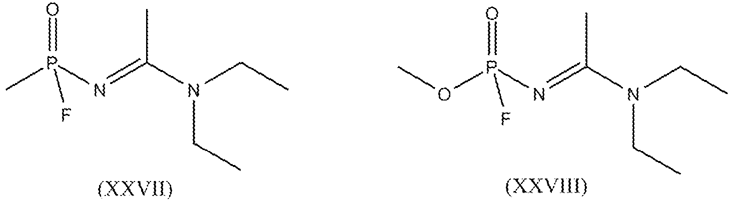

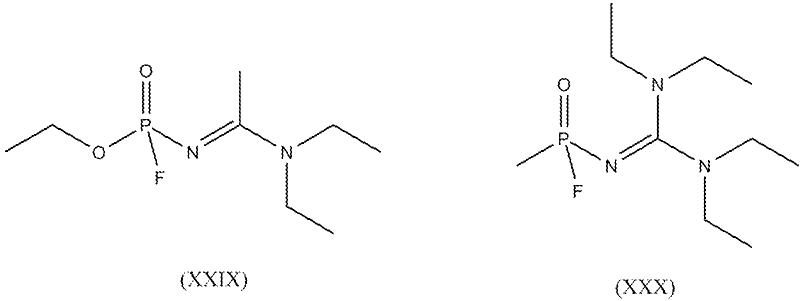

L'invention a enfin également trait à un kit de détection colorimétrique utilisable pour la détection d'un composé chimique comprenant les éléments suivants :
- un système de détection conforme à l'invention et tel que défini ci-dessus ; et
- une échelle colorimétrique permettant de faire la correspondance entre le virage chromatique observé et le composé chimique détecté.

D'autres caractéristiques et avantages de l'invention apparaîtront mieux à la lecture du complément de description qui suit, lequel se rapporte à un exemple de préparation d'un système de détection conforme à l'invention.

Bien entendu, l'exemple qui suit n'est donné qu'à titre d'illustration de l'objet de l'invention et ne constitue en aucun cas une limitation de cet objet.

### EXPOSÉ DÉTAILLÉ DE MODES DE RÉALISATION PARTICULIERS

### EXEMPLE 1

Cet exemple illustre la préparation de différents composés hydrazones conjugués spécifiques utilisables comme indicateur coloré dans le cadre de l'invention, cette préparation étant réalisée par condensation de la fonction hydrazine portée par la 2,4-dinitrophénylhydrazine avec la fonction carbonyle -C=O portée par un réactif carbonylé.

Le protocole général de préparation de ces composés hydrazones est le suivant.

Dans un premier temps, la 2,4-dinitrophénylhydrazine (1,3 éq) est solubilisée à chaud (50°C) à 0,1 M dans l'éthanol, en présence d'acide sulfurique concentré (3 éq). Une solution éthanolique contenant le réactif carbonylé (1 éq) est ensuite ajoutée au goutte à goutte dans la solution comprenant la 2,4-dinitrophénylhydrazine. Le mélange réactionnel est ensuite porté à reflux jusqu'à conversion complète des réactifs. Dans le cas des réactifs dérivés d'aldéhydes, la réaction est quasiment instantanée et l'addition à une température de 50°C est suffisante. L'hydrazone formée n'est que partiellement soluble dans l'éthanol et il se forme à l'issue de la réaction des suspensions de produit dans le milieu réactionnel. Après refroidissement à température ambiante, le mélange réactionnel est évaporé aux 3/4. Le résidu est ensuite partiellement dissous dans un volume d'eau déionisée et traité par une solution basique de NaOH à 1 M. Il se produit une précipitation de l'hydrazone qui est alors filtrée et rincée à l'eau déionisée au minimum 2 fois. Le solide brut est ensuite lavé à chaud dans le solvant adéquat, généralement dans l'éthanol. Il est ensuite séché à l'étuve (80°C) puis sous vide de pompe à palette.

Certaines modalités ont été effectuées en fonction des dérivés carbonylés sélectionnés et sont décrites plus précisément pour chacune des hydrazones.

### a) Modalités précises de préparation du composé 1a

Le paragraphe ci-dessous illustre les modalités précises de préparation du composé 1a répondant à la formule suivante :

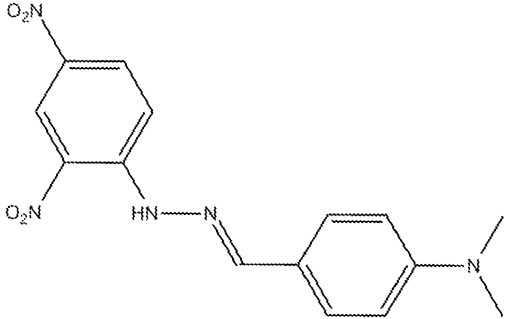

La 2,4-dinitrophénylhydrazine (pureté estimée à 68%, 4,74 mmol, 1,382 g, 1,3 éq) est mise en réaction selon le protocole général de synthèse avec le 4-dimethylaminobenzaldehyde (3,64 mmol, 549,1 mg, 1 éq). Le mélange réactionnel est agité à 50°C pendant 15 min. Le composé hydrazone (1a) est obtenu sous forme d'une poudre de couleur noire (0,98 g, 98%).

Les résultats de spectroscopie IR et de ¹H RMN figurent ci-dessous.

IR: 3302 (NH), 3135 à 2979 (Ar-H), 1604 (C=N), 1583, 1406, 1333, 1281, 1125, 1062, and 698 cm-1;
¹H RMN (DMSO-d₆, 400 MHz): δ 11,57 (s, 1H, N-H), 8,872 (s, 1H, ArDNPH-H), 8,553 (s, 1H, ArDNPH-H), 8,346 (d, 1H, ArDNPH-H), 8,045 (d, 1H, N=CH), 7,623 (d, 2H, Arcarbonyl-H), 6,787 (d, 2H, Arcarbonyl-H), 3,010 (s, 6H, N-CH₃).

### b) Modalités précises de préparation du composé 1b

Le paragraphe ci-dessous illustre les modalités précises de préparation du composé 1b répondant à la formule suivante :

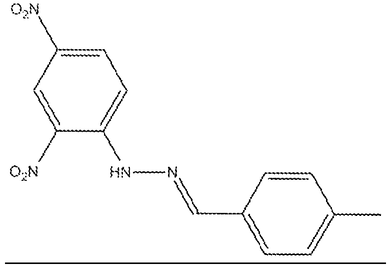

Le p-tolualdéhyde (0,333 mmol, 0,04 mL, 1 éq) sous forme liquide est directement additionné à chaud sur la 2,4-dinitrophénylhydrazine (0,43 mmol, 126,3 mg, 1,3 éq). Le mélange réactionnel est agité à 50°C pendant 15 min. Le composé hydrazone (1b) est obtenu sous forme d'une poudre de couleur orangée (86,9 mg, 86,9%).

Les résultats de spectroscopie ¹H RMN figurent ci-dessous.

¹H RMN (CDCl₃, 400 MHz): δ 11,57 (s, 1H, N-H), 9,149 (d, 1H, ArDNPH-H), 8,35 (dd, 1H, ArDNPH-H), 8,08 (s, 1H, ArDNPH-H), 8,04 (s, 1H, N=CH), 7,667 (d, 2H, Arcarbonyl-H), 7,282 (s, 1H,Ar-H), 2,422 (s, 3H, Ar-CH₃).

### c) Modalités précises de préparation du composé 1c

Le paragraphe ci-dessous illustre les modalités précises de préparation du composé 1c répondant à la formule suivante :

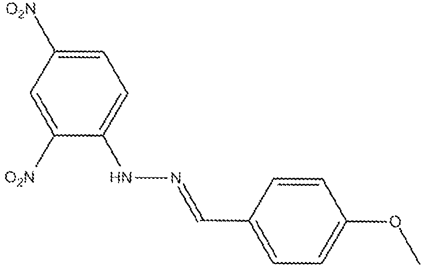

L'anisaldéhyde (0,32 mmol, 0,038 mL, 1 éq) sous forme liquide est directement additionné à chaud sur la 2,4-dinitrophenylhydrazine (0,41 mmol, 119,9 mg, 1,3 éq). Le mélange réactionnel est agité à 50°C pendant 15 min. Le composé hydrazone (1c) est obtenu sous forme d'une poudre de couleur rouge orangée (73,4 mg, 73,4%)

Les résultats de spectroscopie ¹H RMN figurent ci-dessous.

¹H RMN (CDCl₃, 400 MHz): δ 11,57 (s, 1H, N-H), 9,151 (d, 1H, ArDNPH-H), 8,342 (dd, 1H, ArDNPH-H), 8,0615 (s, 1H, ArDNPH-H), 8,04 (s, 1H, N=CH), 7,724 (d, 2H, Arcarbonyl-H), 6,984 (d, 2H, Arcarbonyl-H), 3,882 (s, 3H, Ar-OCH₃)

### d) Modalités précises de préparation du composé 1d

Le paragraphe ci-dessous illustre les modalités précises de préparation du composé 1d répondant à la formule suivante :

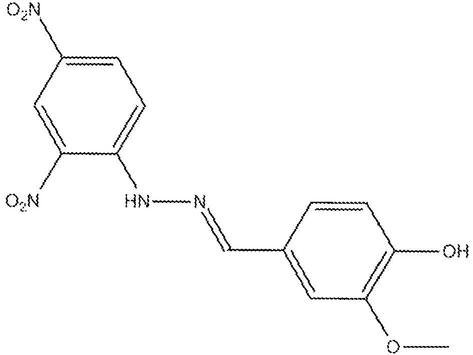

La 2,4-dinitrophénylhydrazine (0,452 mmol, 131,8 mg, 1 éq) est mise en réaction selon le protocole général de synthèse avec la vanilline (0,452 mmol, 69,4 mg, 1 éq). Le mélange réactionnel est agité à 50°C pendant 1h. Dans le cas de cette hydrazone aucun traitement basique n'est réalisé. Le produit est seulement rincé à l'éthanol. Le composé hydrazone (1d) est obtenu sous forme d'une poudre rouge (146,5 mg, 97,5%)

Les résultats de spectroscopie IR et ¹H RMN figurent ci-dessous.

IR (cm⁻¹) v: 3385 (O-H), 3278 (NH), 1622 (C=N), 1515 (NO2), 1418, 1334, 1268 (C-O), 1134, 1062 et 865
¹H RMN (DMSO-d₆, 500 MHz) δ (ppm): 11,59 (s, 1H, N-H), 9,694 (s, 1H, OH), 8,88 (s, 1H), 8,584 (s, 1H, N=CH), 8,35 (d, 1H, Ar-H), 8,10 (d, J = 9,66 Hz, 1H), 7,40 (s, 1H), 7,18 (d, 1H), 6,88 (d, J = 8,1 Hz, 1H), 3,875 (s, 3H, OCH₃)

### e) Modalités précises de préparation du composé 1e

Le paragraphe ci-dessous illustre les modalités précises de préparation du composé 1e répondant à la formule suivante :

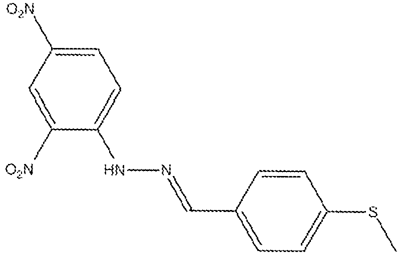

Le 4-méthylthiobenzaldéhyde (0,602 mmol, 82,51 µL, 1 éq) sous forme liquide est directement additionné à chaud sur la 2,4-dinitrophénylhydrazine (0,783 mmol, 0,228 g, 1,3 éq). Le mélange réactionnel est agité à 50°C pendant 2 h. Le composé hydrazone (1e) est obtenu sous forme d'une poudre de couleur rouge orangée (168 mg, 84%)

Les résultats de spectroscopie ¹H RMN figurent ci-dessous.

¹H RMN (CDCl₃, 500 MHz): δ 11,306 (s, 1H, N-H), 9,155 (s, 1H), 8,353 (ddd, 1H), 8,080 (t, 2H), 7,682 (d, 2H), 7,30 (d, 2H), 3,541(s, 3H)

### f) Modalités précises de préparation du composé 1f

Le paragraphe ci-dessous illustre les modalités précises de préparation du composé 1f répondant à la formule suivante :

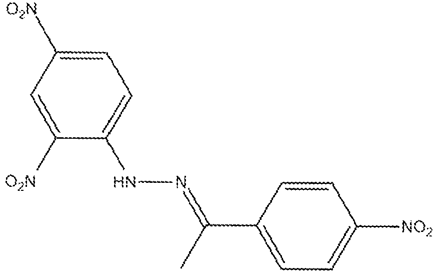

La 2,4-dinitrophénylhydrazine (0,75 mmol, 219,7 mg, 1,3 éq) est mise en réaction selon le protocole général de synthèse avec la 4-nitroacétophénone (0,58 mmol, 96,6 mg, 1 éq). Le mélange réactionnel est agité à 50°C pendant 1h. Le composé hydrazone (1f) est obtenu sous forme d'une poudre jaune orangé (162 mg, 81%).

¹H RMN (CDCl₃, 500 MHz): δ 11,59 (s, 1H, N-H), 9,195 (s, 1H), 8,43 (dd, 1H), 8,315 (d, 2H), 8,142 (d, 1H), 8,02 (d, 2H), 2,518 (s, 3H)

### g) Modalités précises de préparation du composé 2a

Le paragraphe ci-dessous illustre les modalités précises de préparation du composé 2a répondant à la formule suivante :

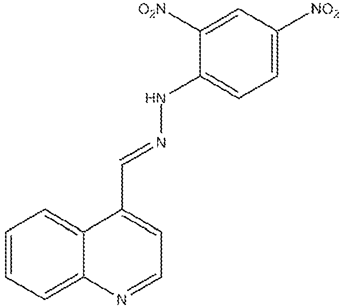

La 2,4-dinitrophénylhydrazine (0,964 mmol, 281,2 mg, 1,3 éq), est mise en réaction selon la procédure générale avec le 4-quinoléine carboxaldéhyde (0,741 mmol, 120,1 mg, 1 éq). Le mélange réactionnel est agité à 50°C pendant 4h. Le composé hydrazone (2a) est obtenu sous forme d'une poudre jaune (255 mg, 100%).

### h) Modalités précises de préparation du compose 2b

Le paragraphe ci-dessous illustre les modalités précises de préparation du composé 2b répondant à la formule suivante :

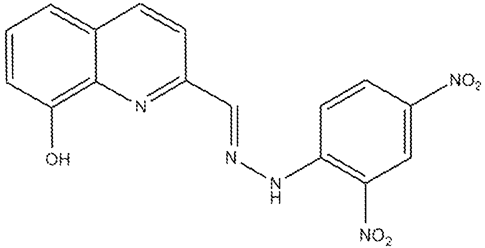

La 2,4-dinitrophénylhydrazine (1,104 mmol, 322,1 mg, 1,3 éq), est mise en réaction selon la procédure générale avec le 8-hydroxy-2-quinoléine carboxaldéhyde (0,849 mmol, 151,5 mg, 1 éq). Le mélange réactionnel est agité à 50°C pendant 2h. Le composé hydrazone (2b) est obtenu sous forme d'une poudre jaune (234,56 mg, 78,2%).

### i) Modalités précises de préparation du composé 3a

Le paragraphe ci-dessous illustre les modalités précises de préparation du composé 3a répondant à la formule suivante :

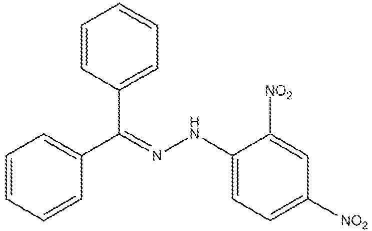

La 2,4-dinitrophénylhydrazine (4,414 mmol, 1,21g, 1,3 éq) est mise en réaction selon le protocole général de synthèse avec la benzophénone (2,76 mmol, 0,508 g, 1 éq). Le mélange réactionnel est porté à reflux pendant 1h et suivi par CCM (toluène : THF 99 :1, Rf=0.67). Le composé hydrazone (3a) est obtenu sous forme d'une poudre orange (0,98g, 98%).

Les résultats de spectroscopie IR et ¹H RMN figurent ci-dessous.

IR: 3302 (NH), 3135 à 2979 (Ar-H), 1604 (C=N), 1583, 1406, 1333, 1281, 1125, 1062, and 698 cm⁻¹
¹H RMN (DMSO-d₆, 400 MHz): δ (ppm) 11,25 (1 H, s, N-H), 8,829 (s, 1H, ArDNPH-H), 8,472 à 8,2531 (dd, 2H, ArDNPH-H), 7,69 (m, 5H, Ar-H), 7,49 (s, 5H, Ar-H)

### j) Modalités précises de préparation du compose 3b

Le paragraphe ci-dessous illustre les modalités précises de préparation du composé 3b répondant à la formule suivante :

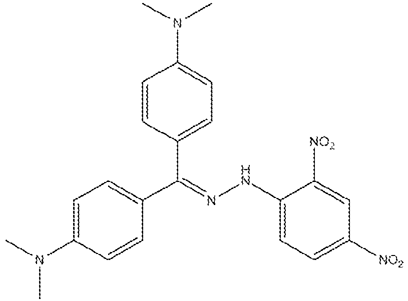

La 2,4-dinitrophénylhydrazine (0,58 mmol, 169,2 mg, 1.3 éq) est mise en réaction dans le méthanol selon le protocole général de synthèse avec la 4,4'-bisdimethylaminobenzophenone (0,45 mmol, 120,9 mg, 1 éq). Le mélange réactionnel est porté à reflux pendant 4 h et suivi par CCM (toluène : THF 98 :2, Rf= 0.3). Le composé hydrazone (3b) est obtenu sous forme d'une poudre pourpre très foncé (162,8 mg, 81,4%).

Les résultats de spectroscopie IR et ¹H RMN figurent ci-dessous.

IR: 3275 (NH), 3000 à 2800 (Ar-H), 1607 (C=N), 1590, 1512, 1328, 1132, 1083, 827 et 741 cm⁻¹
¹H RMN (DMSO-d₆, 400 MHz): δ (ppm) 11,29 (s, 1H, N-H), 8,81 (s, 1H), 8,35 (d, 1H), 8,12 (d, 1H), 7,36 (dd, 2H), 7,05 (dd, 2H), 6,78 (dd, 2H), 6,59 (dd, 2H)

### k) Modalités précises de préparation du compose 3 c

Le paragraphe ci-dessous illustre les modalités précises de préparation du composé 3c répondant à la formule suivante :

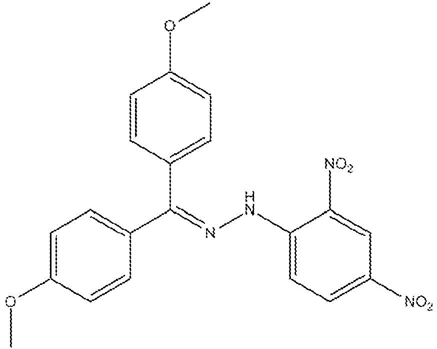

La 2,4 dinitrophénylhydrazine (0,58 mmol, 168,9 mg, 1,3 éq), est mise en réaction dans le méthanol selon le protocole général avec la 4,4'-Dimethoxybenzophenone (0,45 mmol, 110,6 mg, 1 éq). Le mélange réactionnel est porté à reflux pendant 1 h et suivi par CCM (Cyclohexane :AcOEt 70 :30, Rf= 0,62). Le composé hydrazone (3c) est obtenu sous forme d'une poudre rouge légèrement pailletée (162 mg, 81 %)

Les résultats de spectroscopie IR et ¹H RMN figurent ci-dessous.
IR: 3273 (NH), 3000 à 2800 (Ar-H), 1614 (C=N), 1587, 1503, 1334, 1251, 1136, 1025, 1083 et 840 cm⁻¹
¹H RMN (DMSO-d₆, 400 MHz): δ (ppm) 11,56 (s, 1H, N-H), 8,828 (s, 1H, ArDNPH-H), 8,435 (dd, 1H, ArDNPH-H), 8,21 (d, 1H, ArDNPH-H), 7,59 (d, 2H, Ar-H), 7,405 (d, 2H, Ar-H), 7,24 (d, 2H, Ar-H), 7,02 (d, 2H, Ar-H), 3,896 (s, 3H, OCH₃), 3,818 (s, 3H, OCH₃)

### I) Modalités précises de préparation du composé 4

Le paragraphe ci-dessous illustre les modalités précises de préparation du composé 4 répondant à la formule suivante :

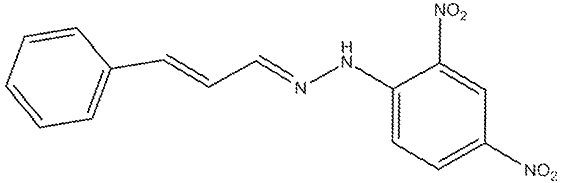

Le trans-cinnamaldéhyde (0,32 mmol, 0,041 mL, 1 éq) sous forme liquide est directement additionné à chaud sur la 2,4-dinitrophénylhydrazine (0,416 mmol, 121,5 mg, 1,3 éq). Le mélange réactionnel est agité à 50°C pendant 15 min. L'hydrazone (1c) est obtenue sous forme d'une poudre de couleur rouge (88,5 mg, 88.5 %)

Les résultats de spectroscopie IR, ¹H RMN et de ¹³C RMN figurent ci-dessous.

IR: 3302 (NH), 3135 à 2979 (Ar-H), 1604 (C=N), 1583, 1406, 1333, 1281, 1125, 1062, and 698 cm⁻¹
¹H RMN (CDCl₃, 400 MHz): δ (ppm) 11,5 (s, 1H, N-H), 9,0143 (s, 1H, ArDNPH-H), 8,356 à 8,326 (dd, 1H, ArDNPH-H), 8,006 à 7,982 (d, 1H, ArDNPH-H), 7,946 (d, 1H, N=CH), 7,52 (d, 2H), 7,425 à 7,36 (m, 3H, Ar-H), 7,03 (m, 2H, C=CH)
¹³C RMN (CDCl₃, 400 MHz) : δ (ppm) 149,84 (HC=N), 141,18 (CAr-NHN), 130,04, 129,49, 128,86, 127,33, 124,07, 123,51, 116,77

### m) Modalités précises de préparation du composé 5a

Le paragraphe ci-dessous illustre les modalités précises de préparation du composé 5a répondant à la formule suivante :

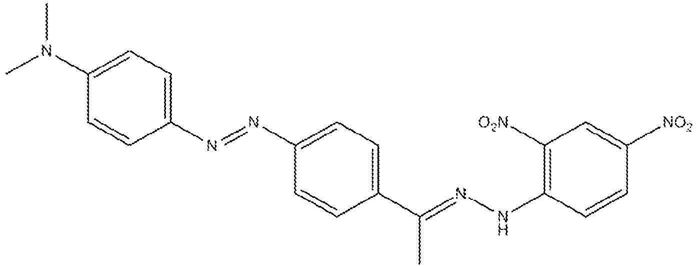

La 2,4-dinitrophénylhydrazine (0,97 mmol, 257 mg, 1,3 éq), est mise en réaction selon la procédure générale avec la 4-(4-diméthylamino)phénylazo)acétophénone (0,75 mmol, 200 mg, 1 éq) en présence de 4 équivalents d'acide sulfurique. Le mélange réactionnel porté à reflux pendant 2h. Le composé hydrazone (5a) est obtenu sous forme d'une poudre rouge brique (267,7 mg, 80%).

### n) Modalités précises de préparation du composé 5b

Le paragraphe ci-dessous illustre les modalités précises de préparation du composé 5b répondant à la formule suivante :

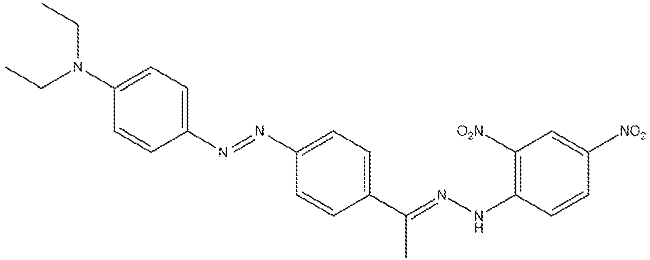

La 2,4-dinitrophénylhydrazine (0,04 mmol, 12,9 mg, 1,3 éq), est mise en réaction selon la procédure générale avec la 4-(4-diéthylamino)phénylazo)acétophénone (0,03 mmol, 10 mg, 1 éq) solubilisée à 0,025 M dans l'éthanol. Le mélange réactionnel porté à reflux pendant 2h. Le composé hydrazone (5b) est obtenu sous forme d'une poudre rouge brique (12,3 mg, 76,4%).

### o) Modalités précises de préparation du composé 6

Le paragraphe ci-dessous illustre les modalités précises de préparation du composé 6 répondant à la formule suivante :

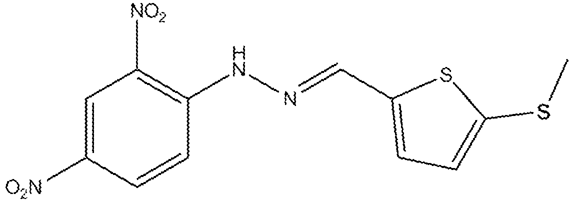

La 2,4-dinitrophénylhydrazine (0,769 mmol, 224,5 mg, 1,3 éq), est mise en réaction selon la procédure générale avec le 5-(méthylthio)thiophène-2-carbaldéhyde (0,592 mmol, 96,4 mg, 1 éq). Le mélange réactionnel est agité à 50°C pendant 2h30. Le composé hydrazone (6) est obtenu sous forme d'une poudre rouge brique (175,14 mg, 87,6%).

Les résultats de spectroscopie ¹H RMN figurent ci-dessous.

¹H RMN (CDCl₃, 400 MHz): δ 11,26 (s, 1H, N-H), 9,137 (d, 1H), 8,3314 à 8,3615 (dd, 1H), 8,1696 (s, 1H), 7,997 à 7,973 (d, 1H), 7,189 (d, 1H), 6,959 (d, 1H), 2,608 (s, 3H)

### p) Modalités précises de préparation du composé 7

Le paragraphe ci-dessous illustre les modalités précises de préparation du composé 7 répondant à la formule suivante :

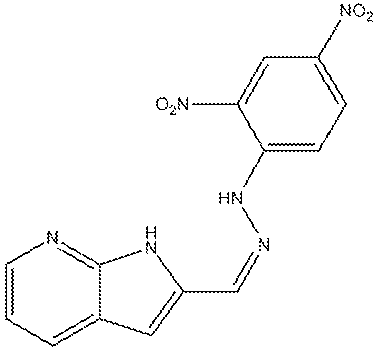

La 2,4-dinitrophénylhydrazine (0,797 mmol, 231,7 mg, 1,3 éq), est mise en réaction selon la procédure générale avec la 7-azaindole-3-carboxaldéhyde (0,613 mmol, 92,4 mg, 1 éq). Le mélange réactionnel est agité à 50°C pendant 15 minutes. Le composé hydrazone (7) est obtenu sous forme d'une poudre rouge brique (152,78 mg, 76,4%).

Les résultats de spectroscopie ¹H RMN figurent ci-dessous.

¹H RMN (DMSO-d₆, 400 MHz): δ 12,311 (s, 1H, N-H), 11,632 (s, 1H, N-H), 8,882 (s, 1H), 8,338 (s, 1H), 8,585 (d, 1H), 8,385 (m, 2H), 8,08 (s, 1H), 8,06 (s, 1H), 7,30 (m, 1H).

### EXEMPLE 2

Dans cet exemple, les composés 1d, 1f, 3c, 1b, 2a, 2b et 6 dont la préparation est exposée dans l'exemple 1 ci-dessus sont déposés respectivement, via une solution comprenant le composé donné (10 mM) dans du diméthylsulfoxyde, sur des supports distincts à base de fibres de verre. Les supports sont ensuite séchés naturellement pour évaporer le diméthylsulfoxyde puis scannés pour constituer des témoins avant exposition à des composés chimiques.

Chacun des supports est ensuite soumis à 13 composés toxiques déposés chacun, à raison de 1,6 µL via une pipette électronique multicanaux, dans des alvéoles individuelles prévues sur le support. Chacun des supports est ensuite scanné après 5 minutes puis après 1 heure d'exposition aux composés toxiques.

Les 13 composés toxiques sont des composés organophosphorés de la série G (Sarin, Soman et Tabun (GA, CAS n°77-81-6), un composé organosphosphoré de la série V (le VX), des vésicants (l'ypérite soufrée (dite HD, CAS n°505-60-2), l'ypérite azotée (dite HN-3, CAS n°555-71-1), la léwisite (dite L1, CAS n°541-25-3), des composés arséniés (la diphénylchlorarsine dit « C1 » et la diphénylcyanoarsine dit « C2 ») et les composés organophosphorés répondant aux formules (XXVII), (XXVIII), (XXIX) et (XXX) suivantes :

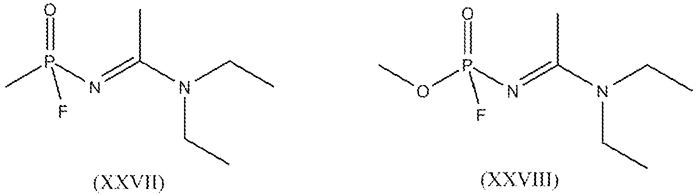

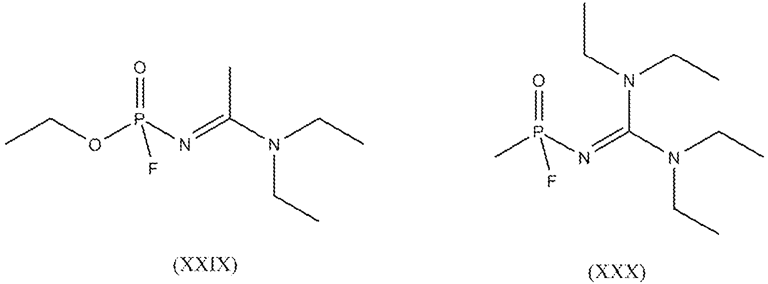

Pour chacun des supports, l'exposition aux composés organophosphorés de formules (XXVII), (XXVIII), (XXIX) et (XXX) engendre un virage de couleur du jaune ou orangé vers pourpre, bleu ou brun et ce de manière instantanée, alors qu'il n'y a pas de virage pour les autres composés.

Cet exemple atteste ainsi de la spécificité de détection des composés organophosphorés de formules (XXVII), (XXVIII), (XXIX) et (XXX) par les composés hydrazones mentionnés ci-dessus.

### EXEMPLE 3

Dans cet exemple, les composés 1f, 1b, 3a, 5a, dont la préparation est exposée dans l'exemple 1 ci-dessus, et des composés de formules (V) et (XI) suivantes : 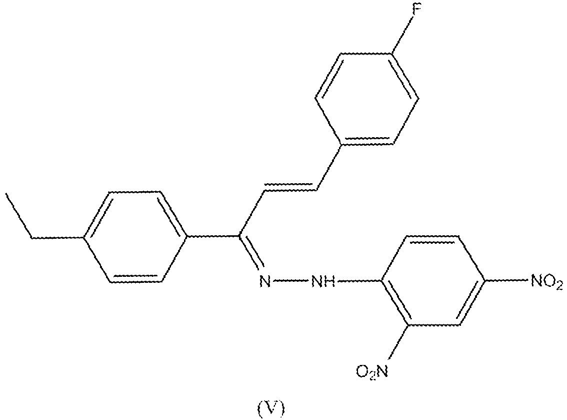 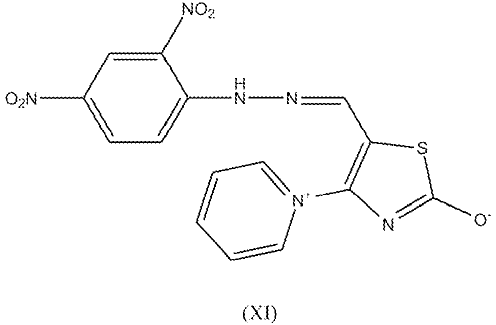 sont déposés respectivement, via une solution comprenant le composé donné (10 mM) dans du diméthylsulfoxyde, sur des supports distincts à base de fibres de verre. Les supports sont ensuite séchés naturellement pour évaporer le diméthylsulfoxyde puis scannés pour constituer des témoins avant exposition à des composés chimiques.

Chacun des supports est ensuite soumis à 4 composés toxiques déposés chacun, à raison de 1,6 µL *via* une pipette électronique multicanaux, dans des alvéoles individuelles prévues sur le support. Chacun des supports est ensuite scanné après 5 minutes puis après 1 heure d'exposition aux composés toxiques.

Les 4 composés toxiques répondent aux formules (XXVII), (XXVIII), (XXIX) et (XXX) suivantes :

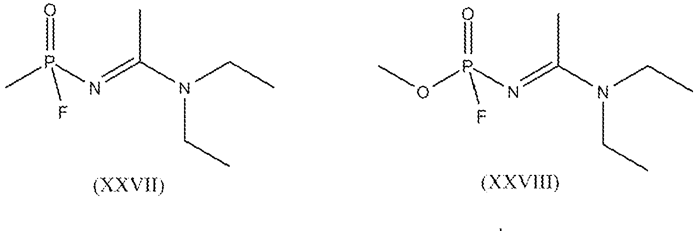

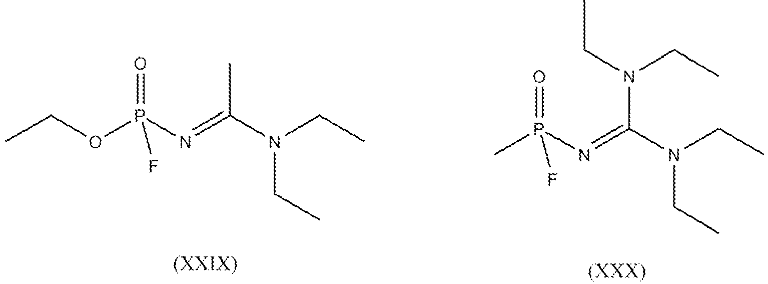

La mise en contact des 4 agents neurotoxiques organophosphorés susmentionnés avec les supports en fibres de verre, sur lesquels ont été déposés les composés hydrazones mentionnés ci-dessus, entraîne un virage de couleur de ceux-ci, attestant ainsi de l'efficacité des supports conformes à l'invention pour la détection de ce type spécifique de composés organophosphorés.

### EXEMPLE 4

Dans cet exemple, les composés 1a, 1b, 1c, 1d, 1e, 1f, 2a, 2b, 3a, 3b, 3c, 4, 5a, 5b et 6 dont la préparation est exposée dans l'exemple 1 ci-dessus, les composés de formules (V) et (XI) telles que définies ci-dessus et les composés de formules suivantes : 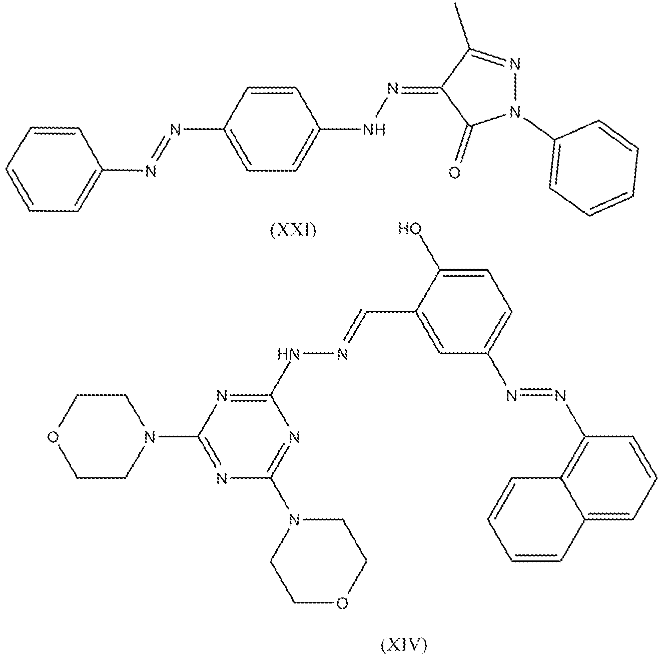 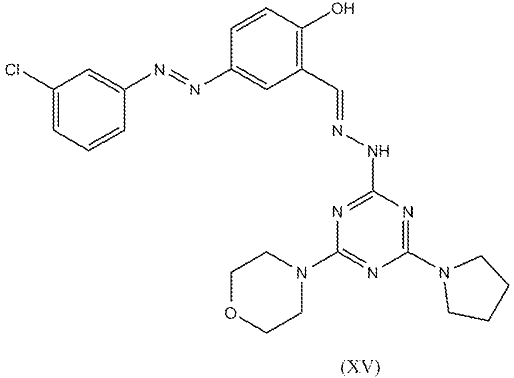 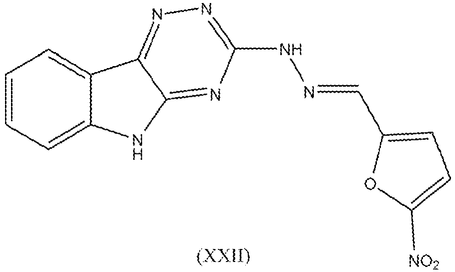 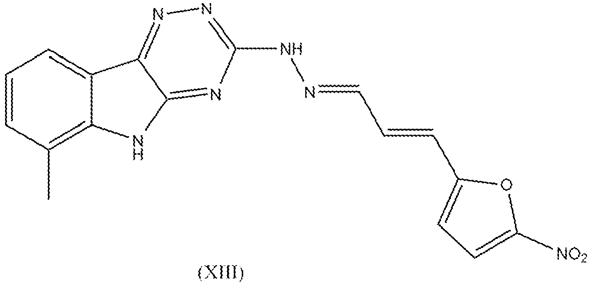 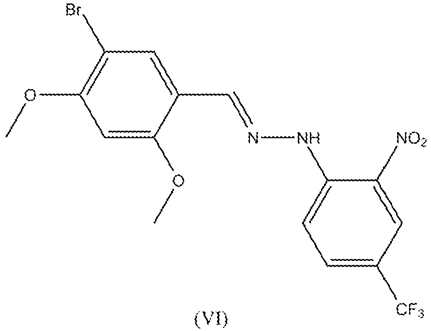 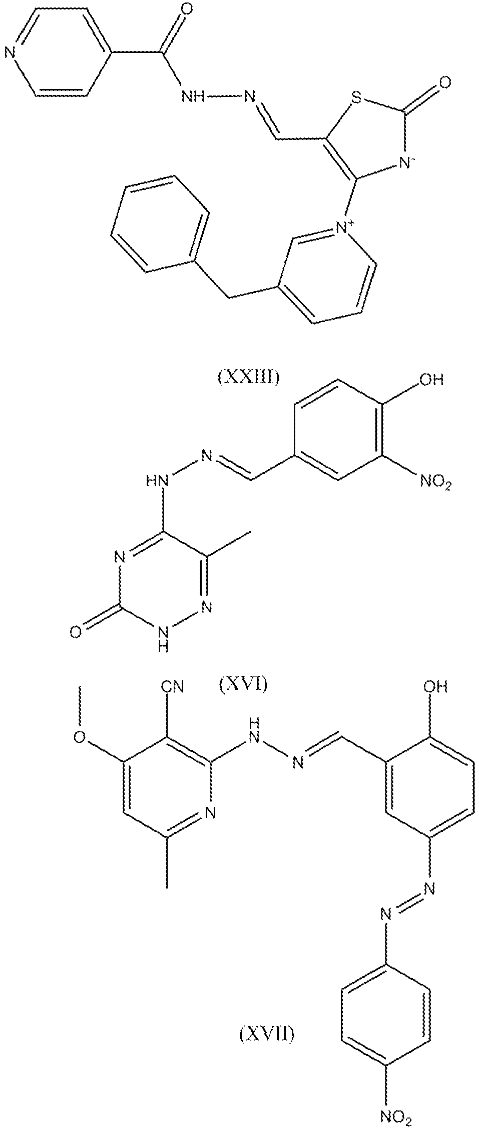 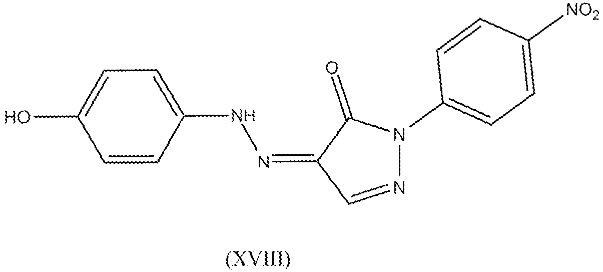 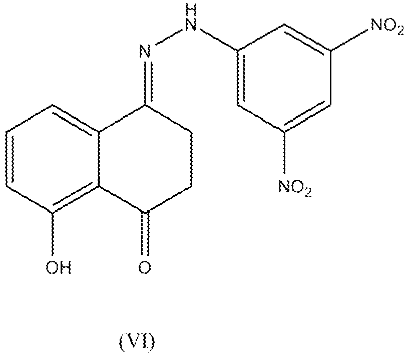 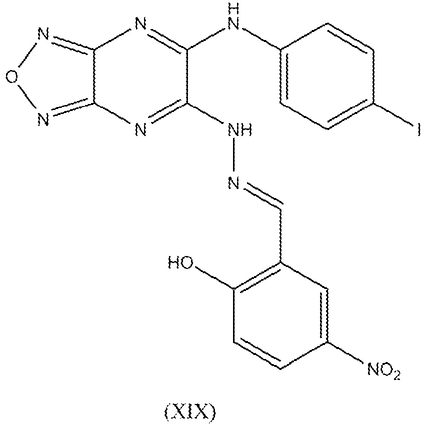 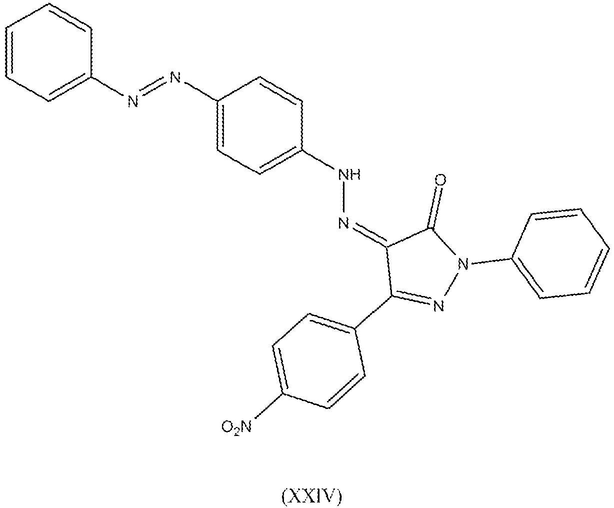 sont déposés respectivement, via une solution comprenant le composé donné (10 mM) dans du diméthylsulfoxyde, sur des supports distincts à base de fibres de verre. Les supports sont ensuite séchés naturellement pour évaporer le diméthylsulfoxyde puis scannés pour constituer des témoins avant exposition à des composés chimiques.

Chacun des supports est ensuite soumis à 4 composés toxiques déposés chacun, à raison de 1,6 µL *via* une pipette électronique multicanaux, dans des alvéoles individuelles prévues sur le support. Chacun des supports est ensuite scanné après 5 minutes puis après 1 heure d'exposition aux composés toxiques. Les 4 composés toxiques sont ceux définis à l'exemple 3 ci-dessous.

La mise en contact des 4 agents neurotoxiques organophosphorés susmentionnés avec les supports en fibres de verre, sur lesquels ont été déposés les composés hydrazones mentionnés ci-dessus, entraîne un virage de couleur de ceux-ci, attestant ainsi de l'efficacité des supports conformes à l'invention pour la détection de ce type spécifique de composés organophosphorés.

### EXEMPLE 5

Dans cet exemple, les composés 1a, 1c, 1e, 1f, 2a, 2b, 3b, 4, dont la préparation est exposée dans l'exemple 1 ci-dessus, les composés de formules (XXI), (VI), (XIX) dont les formules sont définies à l'exemple 4 ci-dessus et le composé de formule (XX) suivante : 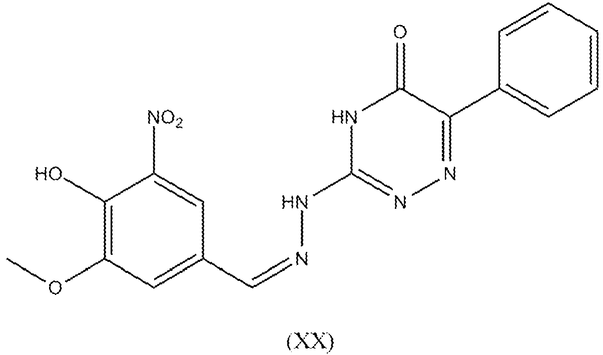 sont déposés respectivement sur des supports distincts en papier de chromatographie, lesdits supports étant soumis aux quatre agents neurotoxiques organophosphorés tels que définis à l'exemple 3 ci-dessus.

La mise en contact des 4 agents neurotoxiques organophosphorés susmentionnés avec les supports en papier de chromatographie, sur lesquels ont été déposés les composés hydrazones mentionnés ci-dessus, entraîne un virage de couleur de ceux-ci, attestant ainsi de l'efficacité des supports conformes à l'invention pour la détection de ce type spécifique de composés organophosphorés.

## Revendications

1. Système de détection colorimétrique utilisable pour la détection d'un composé chimique comprenant un support comprenant un indicateur coloré dudit composé chimique à détecter, **caractérisé en ce que** l'indicateur coloré est un composé hydrazone conjugué choisi parmi :
a) les composés hydrazones conjugués comprenant au moins un groupe cyclique lié à l'atome de carbone porteur de la double liaison de la fonction hydrazone et comprenant un groupe aromatique porteur d'au moins un groupe électroattracteur lié à l'atome d'azote de la fonction hydrazone ;
b) les composés hydrazones conjugués comprenant au moins un groupe éthylénique lié à l'atome de carbone porteur de la double liaison de la fonction hydrazone et comprenant, en outre, un groupe aromatique porteur d'au moins un groupe électroattracteur ;
c) les composés hydrazones conjugués comprenant au moins un groupe aromatique porteur d'un groupe -OH lié à l'atome d'azote de la fonction hydrazone ou à l'atome de carbone porteur de la double liaison de la fonction hydrazone ;
d) les composés hydrazones conjugués comprenant un groupe hétéroaromatique lié à l'atome d'azote de la fonction hydrazone et un autre groupe hétéroaromatique lié à l'atome de carbone porteur de la double liaison de la fonction hydrazone ou comprend un groupe diazoaromatique lié à l'atome d'azote de la fonction hydrazone et un groupe hétéroaromatique englobant l'atome de carbone porteur de la double liaison de la fonction hydrazone.

2. Système de détection colorimétrique selon la revendication 1, dans lequel, lorsque le composé hydrazone conjugué est un composé de la catégorie a), le ou les groupes cycliques liés à l'atome de carbone porteur de la double liaison de la fonction hydrazone sont des groupes cycliques carbonés et le groupe aromatique porteur d'au moins un groupe électroattracteur est un groupe aromatique porteur d'au moins un groupe NO₂.

3. Système de détection colorimétrique selon l'une quelconque des revendications précédentes, dans lequel le composé hydrazone conjugué répond à l'une des formules (II), (III), (IV), (V), (VI) et (VI') suivantes : 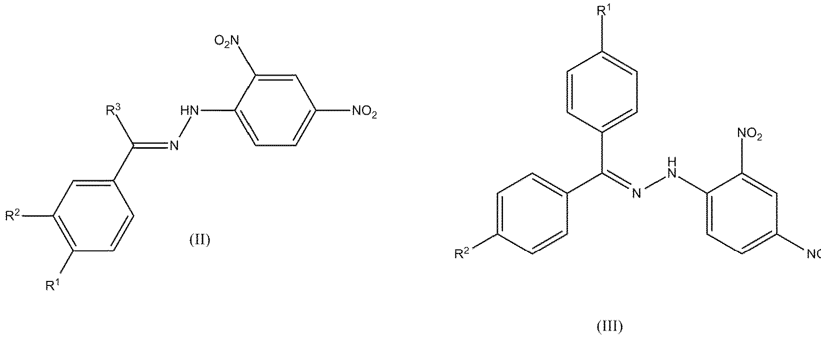 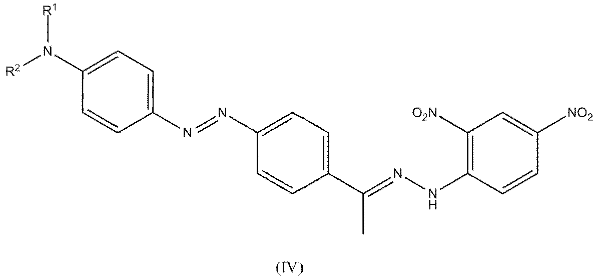 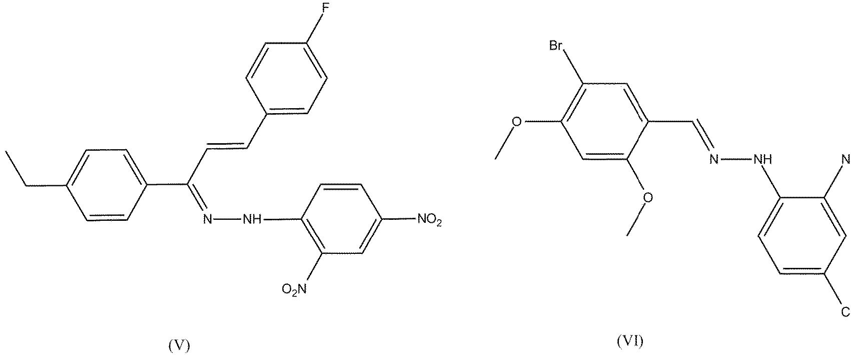 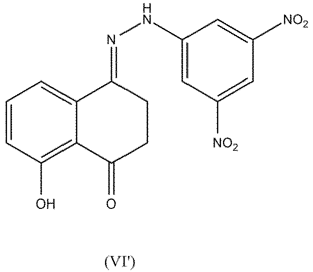 pour lesquelles :
- pour la formule (II), R¹ représente -N(CH₃)₂, -CH₃, -OCH₃, -OH, -SCH₃ ou - NO₂ ; R² représente -H ou -OCH₃ et R³ représente -H ou -CH₃;
- pour la formule (III), R¹ et R² représentent, indépendamment l'un de l'autre, -H, -N(CH₃)₂, -OCH₃ ou -NO₂ ;
- pour la formule (IV), R¹ et R² représentent, indépendamment l'un de l'autre, -CH₃ ou -CH₂-CH₃.

4. Système de détection colorimétrique selon la revendication 3, dans lequel, lorsque le composé hydrazone conjugué est un composé de formule (II), il est :
- le composé pour lequel R¹ représente -N(CH₃)₂, R² représente -H et R³ représente H ;
- le composé pour lequel R¹ représente -CH₃, R² représente H et R³ représente H ;
- le composé pour lequel R¹ représente -OCH₃, R² représente -H et R³ représente H ;
- le composé pour lequel R¹ représente -OH, R² représente -OCH₃ et R³ représente H ;
- le composé pour lequel R¹ représente -SCH₃, R² représente -H et R³ représente H ; ou
- le composé pour lequel R¹ représente -NO₂, R² représente -H et R³ représente -CH₃.

5. Système de détection colorimétrique selon la revendication 3, dans lequel, lorsque le composé hydrazone conjugué est un composé de formule (III), il est :
- le composé pour lequel R¹ représente -H et R² représente -H ;
- le composé pour lequel R¹ représente -N(CH₃)₂ et R² représente -N(CH₃)₂; ou
- le composé pour lequel R¹ représente -OCH₃ et R² représente OCH₃.

6. Système de détection colorimétrique selon la revendication 3, dans lequel, lorsque le composé hydrazone conjugué est un composé de formule (IV), il est :
- le composé pour lequel R¹ représente -CH₃ et R² représente -CH₃ ; ou
- le composé pour lequel R¹ représente -CH₂-CH₃ et R² représente -CH₂-CH₃.

7. Système de détection colorimétrique selon la revendication 1, dans lequel, lorsque le composé hydrazone conjugué est un composé de la catégorie a), le ou les groupes cycliques liés à l'atome de carbone porteur de la double liaison de la fonction hydrazone sont des groupes hétéroaromatiques et le groupe aromatique porteur d'au moins un groupe électroattracteur est un groupe aromatique porteur d'au moins un groupe NO₂.

8. Système de détection colorimétrique selon la revendication 7, dans lequel le composé hydrazone conjugué répond à l'une des formules (VII), (VIII), (IX), (X) et (XI) suivantes : 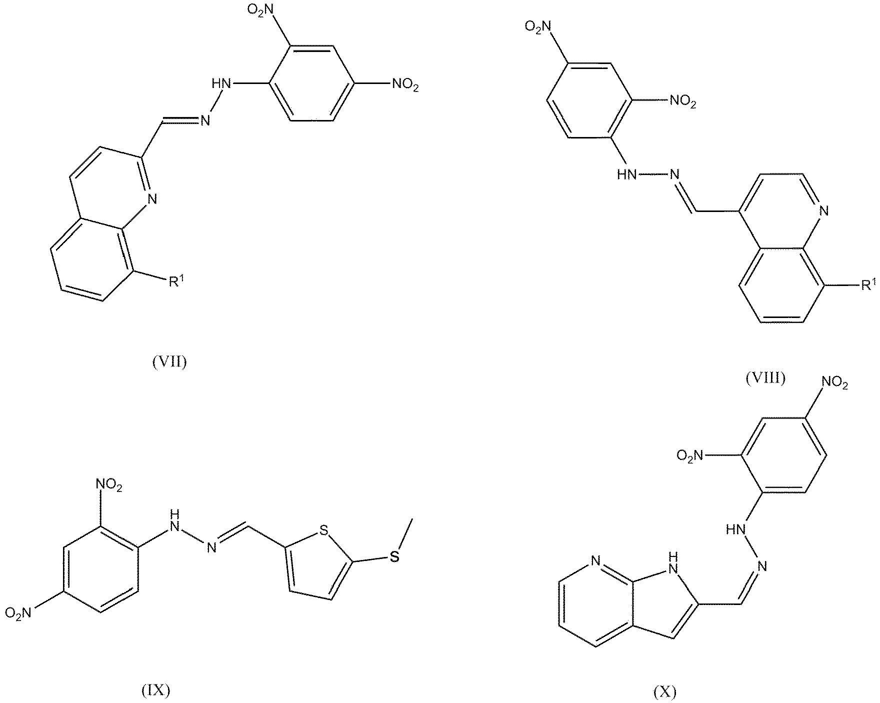 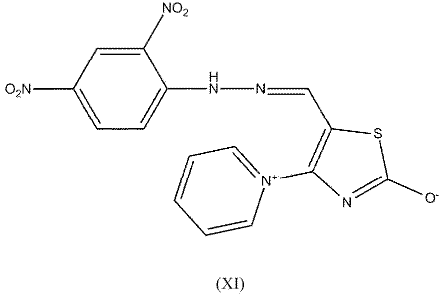 pour lesquelles :
- pour la formule (VII), R¹ représente -H ou -OH;
- pour la formule (VIII), R¹ représente -H ou -OH.

9. Système de détection colorimétrique selon la revendication 1, dans lequel, lorsque le composé hydrazone conjugué est un composé de la catégorie b), il répond à l'une des formules (XII) ou (XIII) suivantes :

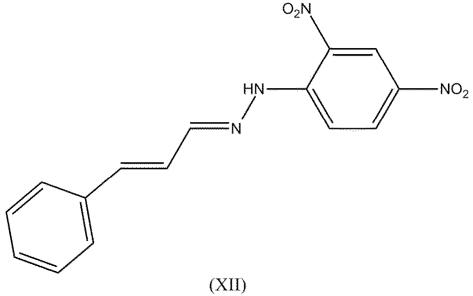

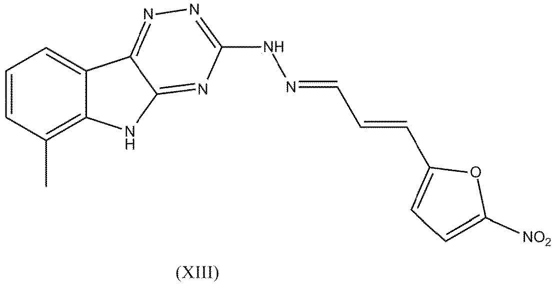

10. Système de détection colorimétrique selon la revendication 1, dans lequel, lorsque le composé hydrazone conjugué est un composé de la catégorie c), il répond à l'une des formules (XIV), (XV), (XVI), (XVII), (XVIII), (XIX) et (XX) suivantes :

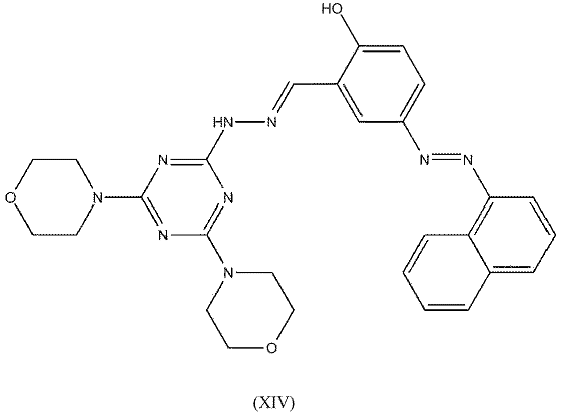

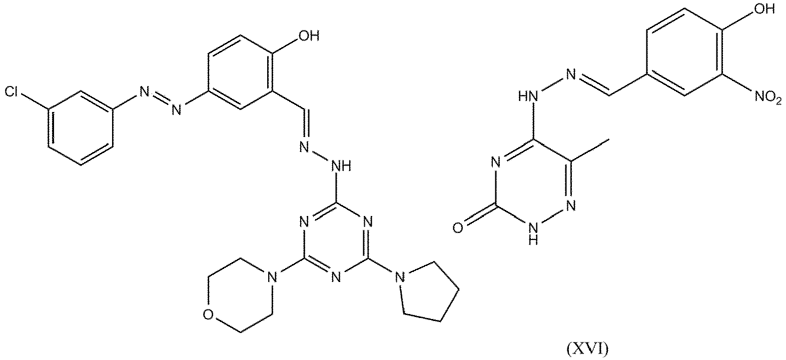

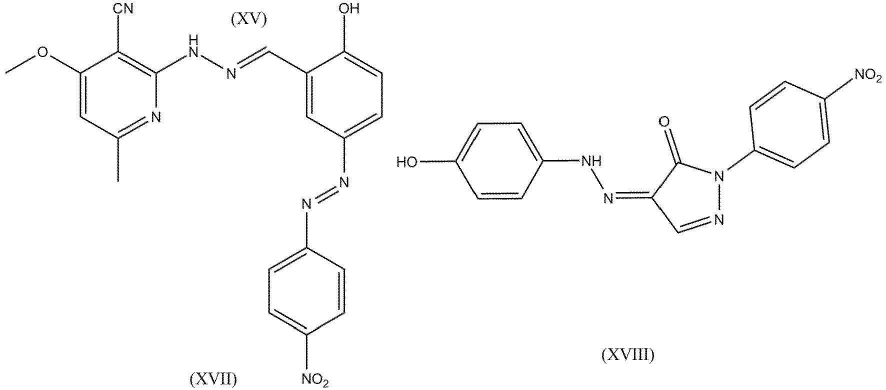

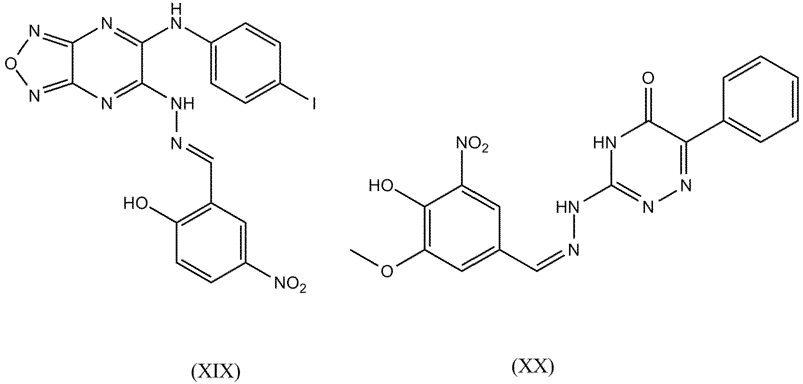

11. Système de détection colorimétrique selon la revendication 1, dans lequel, lorsque le composé hydrazone conjugué est un composé de la catégorie d), il répond à l'une des formules (XXI), (XXII), (XXIII) et (XXIV) suivantes :

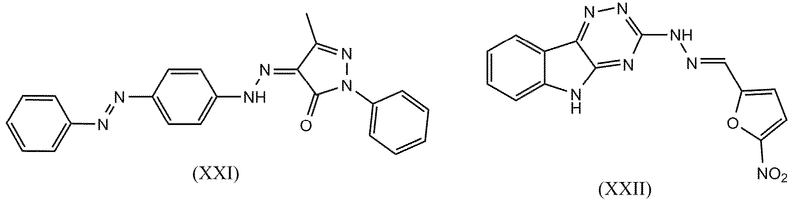

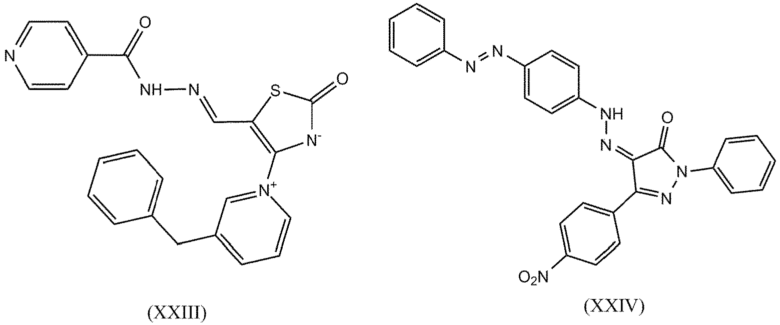

12. Système de détection colorimétrique selon l'une quelconque des revendications précédentes, dans lequel le support comprend des fibres de verre ou est un support en papier, de préférence comprenant des fibres de verre.

13. Composé hydrazone répondant à l'une des formules (5a), (5b) et (X) suivantes :

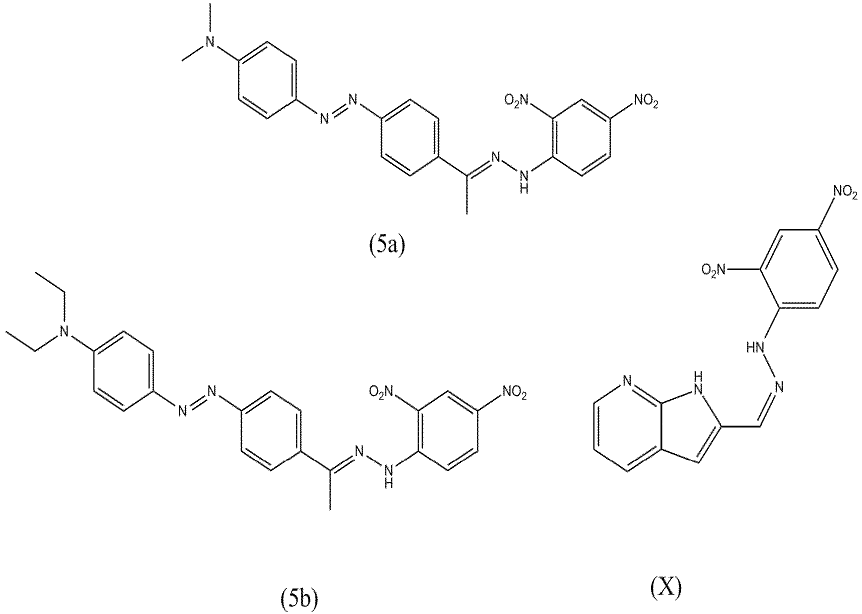

14. Procédé de détection de la présence ou de l'absence d'un composé chimique comprenant les étapes suivantes :
- une étape de mise en contact du ou des milieux, dont on veut détecter la présence ou l'absence dudit composé chimique avec le système de détection tel que défini selon l'une quelconque des revendications 1 à 12 ;
- une étape de déduction, en fonction du ou des éventuels virages chromatiques observés, de la présence ou de l'absence dudit composé chimique.

15. Procédé selon la revendication 14, qui est un procédé de détection de la présence ou de l'absence d'un composé chimique choisi parmi les composés toxiques de guerre, les composés toxiques industriels, les pesticides.

16. Procédé selon la revendication 15, qui est un procédé de détection de la présence ou de l'absence d'un composé chimique répondant à l'une des formules (XXV) et (XXVI) suivantes : 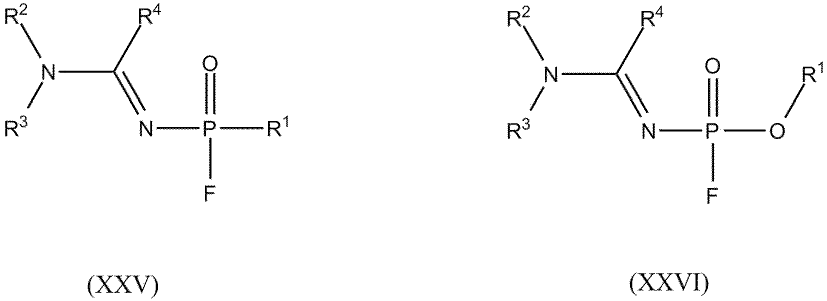 dans lesquelles :
- R¹ représente H ou un groupe alkyle, éventuellement cyclique, pouvant comprendre jusqu'à 10 atomes de carbone ;
- R² et R³ représentent, indépendamment l'un de l'autre, un groupe alkyle, éventuellement cyclique, pouvant comprendre jusqu'à 10 atomes de carbone ;
- R⁴ représente H, un groupe alkyle, éventuellement cyclique, pouvant comprendre jusqu'à 10 atomes de carbone ou un groupe amino.

17. Kit de détection colorimétrique utilisable pour la détection d'un composé chimique comprenant les éléments suivants :
- un système de détection tel que défini selon l'une quelconque des revendications 1 à 12; et
- une échelle colorimétrique permettant de faire la correspondance entre le virage chromatique observé et le composé chimique détecté.

## Patentansprüche

1. Kolorimetrisches Detektionssystem, das zum Nachweis einer chemischen Verbindung verwendbar ist, umfassend einen Träger, der einen Farbindikator der nachzuweisenden chemischen Verbindung umfasst, **dadurch gekennzeichnet, dass** der Farbindikator eine konjugierte Hydrazonverbindung ist, die ausgewählt ist aus:
a) den konjugierten Hydrazonverbindungen, die mindestens eine zyklische Gruppe umfassen, die mit dem Kohlenstoffatom verbunden ist, das die Doppelbindung der Hydrazonfunktion trägt und eine aromatische Gruppe umfassen, die mindestens eine Elektroanziehungsgruppe trägt, die mit dem Stickstoffatom der Hydrazonfunktion verbunden ist;
b) den konjugierten Hydrozonverbindungen, die mindestens eine Ethylengruppe umfassen, die mit dem Kohlenstoffatom verbunden ist, das die Doppelbindung der Hydrozonfunktion trägt und ferner eine aromatische Gruppe umfassen, die mindestens eine Elektroanziehungsgruppe trägt;
c) den konjugierten Hydrazonverbindungen, die mindestens eine aromatische Gruppe umfassen, die eine -OH-Gruppe trägt, die mit dem Stickstoffatom der Hydrazonfunktion oder mit dem Kohlenstoffatom verbunden ist, das die Doppelbindung der Hydrazonfunktion trägt;
d) den konjugierten Hydrazonverbindungen, die eine heteroaromatische Gruppe umfassen, die mit dem Stickstoffatom der Hydrazonfunktion verbunden ist, und eine andere heteroaromatische Gruppe, die mit dem Kohlenstoffatom verbunden ist, das die Doppelbindung der Hydrazonfunktion trägt, oder eine diazoaromatische Gruppe umfasst, die mit dem Stickstoffatom der Hydrazonfunktion verbunden ist und eine heteroaromatische Gruppe, die das Kohlenstoffatom einschließt, das die Doppelbindung der Hydrazonfunktion trägt.

2. Kolorimetrisches Detektionssystem nach Anspruch 1, wobei, wenn die konjugierte Hydrazonverbindung eine Verbindung der Kategorie a) ist, die zyklische(n) Gruppe(n), die mit dem Kohlenstoffatom verbunden ist/sind, das die Doppelbindung der Hydrazonfunktion trägt, kohlenstoffhaltige zyklische Gruppen sind und die aromatische Gruppe, die mindestens eine Elektroanziehungsgruppe trägt, eine aromatische Gruppe ist, die mindestens eine NO₂-Gruppe trägt.

3. Kolorimetrisches Detektionssystem nach einem der vorhergehenden Ansprüche, wobei die konjugierte Hydrazonverbindung einer der folgenden Formeln (II), (III), (IV), (V), (VI) und (VI') entspricht: 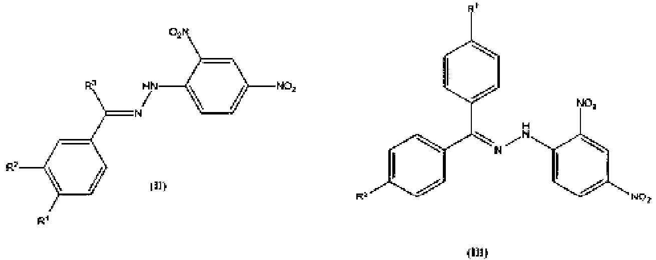 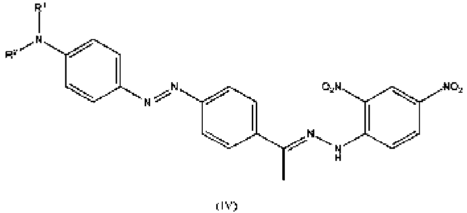 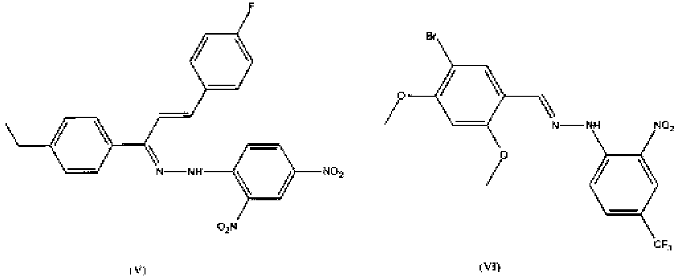 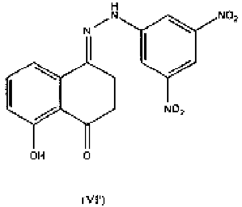 in denen:
- für die Formel (II) R¹ -N(CH₃) ₂, -CH₃, -OCH₃, -OH, -SCH₃ oder -NO₂ darstellt; R² -H oder -OCH₃ darstellt und R³ -H oder -CH₃ darstellt;
- für die Formel (III) R¹ und R² unabhängig voneinander -H, -N(CH₃) ₂, -OCH₃ oder -NO₂ darstellen;
- für die Formel (IV) R¹ und R² unabhängig voneinander -CH₃ oder -CH₂-CH₃ darstellen.

4. Kolorimetrisches Detektionssystem nach Anspruch 3, wobei, wenn die konjugierte Hydrazonverbindung eine Verbindung der Formel (II) ist, gilt:
- die Verbindung, für die R¹ -N(CH₃)₂ darstellt, R² -H darstellt und R³ H darstellt;
- die Verbindung, für die R¹ -CH₃ darstellt, R² H darstellt und R³ H darstellt;
- die Verbindung, für die R¹ -OCH₃ darstellt, R² -H darstellt und R³ H darstellt;
- die Verbindung, für die R¹ -OH darstellt, R² -OCH₃ darstellt und R³ H darstellt;
- die Verbindung, für die R¹ -SCH₃ darstellt, R²-H darstellt und R³ H darstellt; oder
- die Verbindung, für die R¹ -NO₂ darstellt, R² -H darstellt und R³ -CH₃ darstellt.

5. Kolorimetrisches Detektionssystem nach Anspruch 3, wobei, wenn die konjugierte Hydrazonverbindung eine Verbindung der Formel (III), gilt:
- die Verbindung, für die R¹ -H darstellt und R² -H darstellt;
- die Verbindung, für die R¹ -N(CH₃)₂ darstellt und R² -N(CH₃)₂ darstellt; oder
- die Verbindung, für die R¹ -OCH₃ darstellt und R² OCH₃ darstellt.

6. Kolorimetrisches Detektionssystem nach Anspruch 3, wobei, wenn die konjugierte Hydrazonverbindung eine Verbindung der Formel (IV), gilt:
- die Verbindung, für die R¹ -CH₃ darstellt und R² -CH₃ darstellt; oder
- die Verbindung, für die R¹ -CH₂-CH₃ darstellt und R² -CH₂-CH₃ darstellt.

7. Kolorimetrisches Detektionssystem nach Anspruch 1, wobei, wenn die konjugierte Hydrazonverbindung eine Verbindung der Kategorie a) ist, die zyklische(n) Gruppe(n), die mit dem Kohlenstoffatom verbunden ist/sind, das die Doppelbindung der Hydrazonfunktion trägt, heteroaromatische Gruppen sind und die aromatische Gruppe, die mindestens eine Elektroanziehungsgruppe trägt, eine aromatische Gruppe ist, die mindestens eine NO₂-Gruppe trägt.

8. Kolorimetrisches Detektionssystem nach Anspruch 7, wobei die konjugierte Hydrazonverbindung einer der folgenden Formeln (VII), (VIII), (IX), (X) und (XI) entspricht: 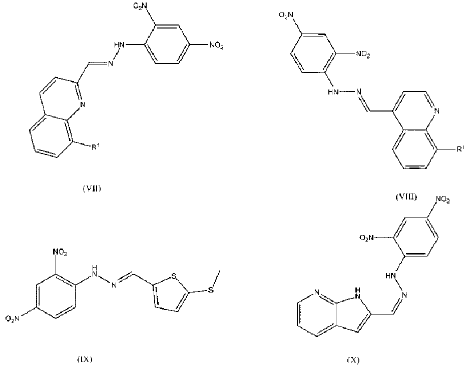 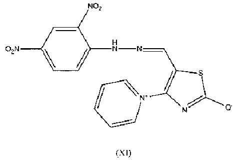 in denen:
- für die Formel (VII) R¹ -H oder -OH darstellt;
- für die Formel (VIII) R¹ -H oder -OH darstellt.

9. Kolorimetrisches Detektionssystem nach Anspruch 1, wobei, wenn die konjugierte Hydrazonverbindung eine Verbindung der Kategorie b) ist, sie einer der folgenden Formeln (XII) oder (XIII) entspricht:

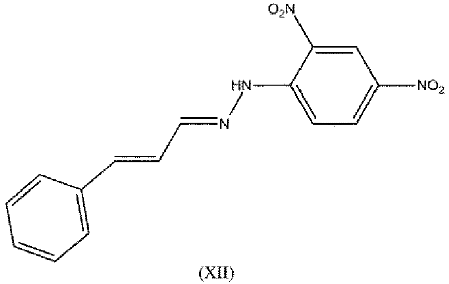

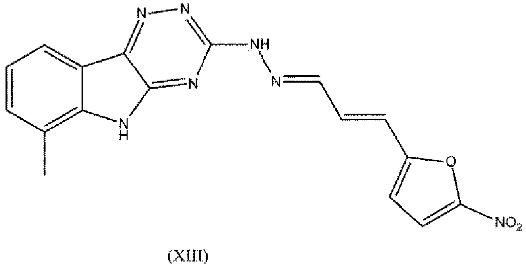

10. Kolorimetrisches Detektionssystem nach Anspruch 1, wobei, wenn die konjugierte Hydrazonverbindung eine Verbindung der Kategorie c) ist, sie einer der folgenden Formeln (XIV), (XV), (XVI), (XVII), (XVIII), (XIX) und (XX) entspricht:

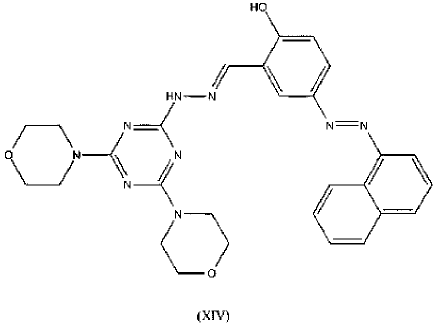

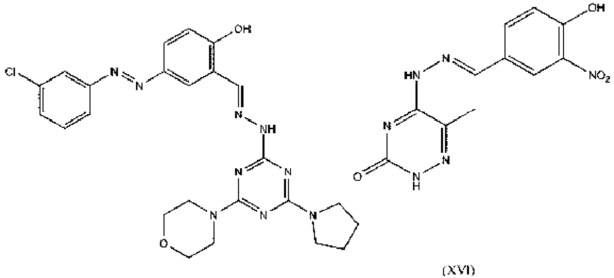

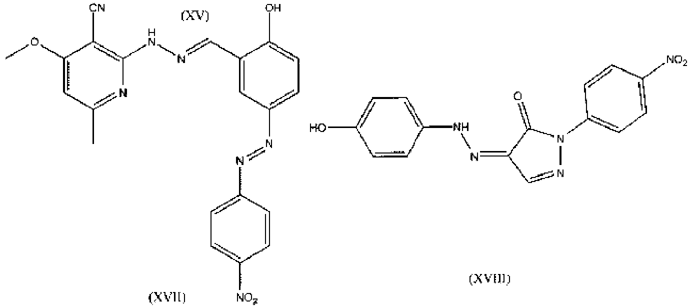

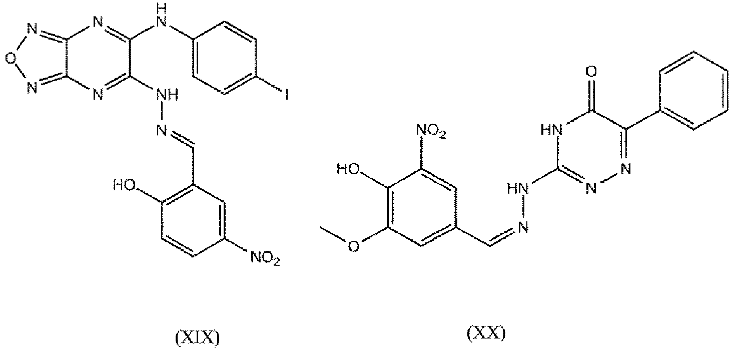

11. Kolorimetrisches Detektionssystem nach Anspruch 1, wobei, wenn die konjugierte Hydrazonverbindung eine Verbindung der Kategorie d) ist, sie einer der folgenden Formeln (XXI), (XXII), (XXIII) und (XXIV) entspricht:

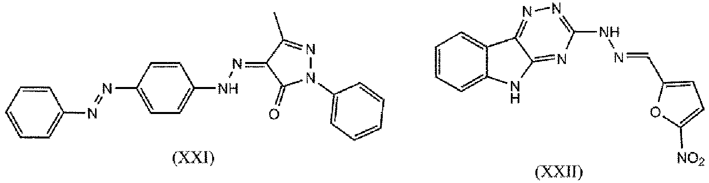

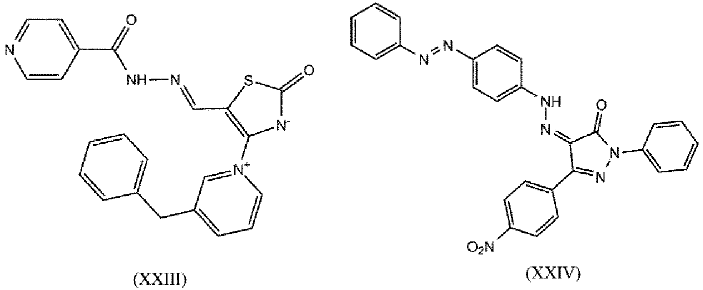

12. Kolorimetrisches Detektionssystem nach einem der vorhergehenden Ansprüche, wobei der Träger Glasfasern umfasst oder ein Papierträger ist, der vorzugsweise Glasfasern umfasst.

13. Hydrazonverbindung, die einer der folgenden Formeln (5a), (5b) und (X) entspricht:

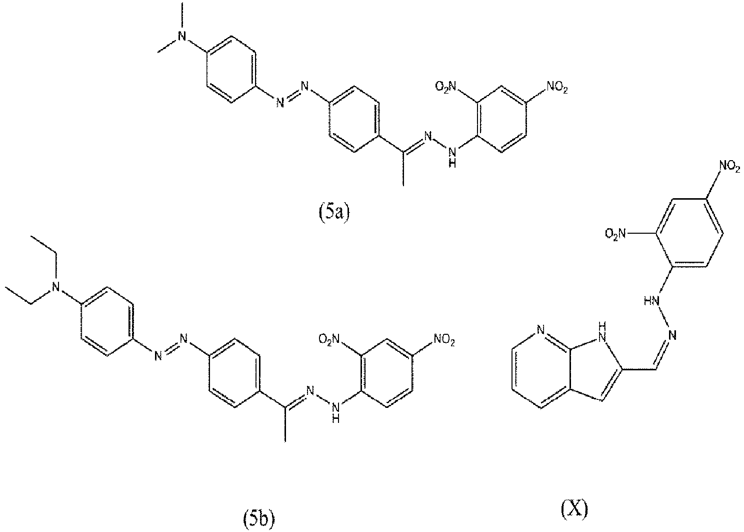

14. Verfahren zum Nachweis des Vorhandenseins oder des Nicht-Vorhandenseins einer chemischen Verbindung, das die folgenden Schritte umfasst:
- einen Schritt des Inkontaktbringens des oder der Medien, bei denen man das Vorhandensein oder Nicht-Vorhandensein der chemischen Verbindung mit dem Detektionssystem nach einem der Ansprüche 1 bis 12 nachweisen will;
- einen Schritt des Schlussfolgerns, in Abhängigkeit von der oder den eventuell beobachteten Farbkurven, des Vorhandenseins oder des Nicht-Vorhandenseins der chemischen Verbindung.

15. Verfahren nach Anspruch 14, das ein Verfahren zum Nachweis des Vorhandenseins oder des Nicht-Vorhandenseins einer chemischen Verbindung ist, die aus toxischen Kriegsverbindungen, toxischen Industrieverbindungen, Pestiziden ausgewählt ist.

16. Verfahren nach Anspruch 15, das ein Verfahren zum Nachweis des Vorhandenseins oder des Nicht-Vorhandenseins einer chemischen Verbindung ist, die einer der Formeln (XXV) und (XXVI) entspricht: 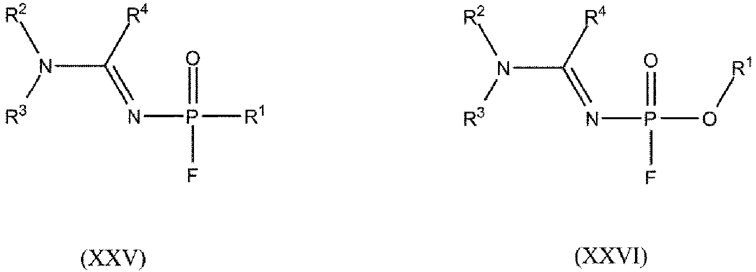 in denen:
- R¹ H oder eine eventuell zyklische Alkylgruppe darstellt, die bis zu 10 Kohlenstoffatomen darstellt;
- R² und R³ unabhängig voneinander eine eventuell zyklische Alkylgruppe darstellen, die bis zu 10 Kohlenstoffatomen darstellen;
- R⁴ H, eine eventuell zyklische Alkylgruppe darstellt, die bis zu 10 Kohlenstoffatomen oder eine Aminogruppe darstellt.

17. Kolorimetrisches Detektionskit, das zum Nachweis einer chemischen Verbindung verwendbar ist, umfassend die folgenden Elemente:
- ein Detektionssystem nach einem der Ansprüche 1 bis 12; und
- eine kolorimetrische Skala, die die Zuordnung der erkannten chemischen Verbindung zu der beobachtete Farbkurve gestattet.

## Claims

1. Colorimetric detection system usable for detecting a chemical compound comprising a carrier comprising a coloured indicator of said chemical compound to be detected, **characterised in that** the coloured indicator is a conjugated hydrazone compound chosen from:
a) conjugated hydrazone compounds comprising at least one cyclic group bonded to the carbon atom carrying the double bond of the hydrazone function and comprising an aromatic group carrying at least one electro-attractive group bonded to the nitrogen atom of the hydrazone function;
b) conjugated hydrazone compounds comprising at least one ethylene group bonded to the carbon atom carrying the double bond of the hydrazone function and further comprising an aromatic group carrying at least one electro-attractive group;
c) conjugated hydrazone compounds comprising at least one aromatic group carrying an -OH group bonded to the nitrogen atom of the hydrazone function or to the carbon atom carrying the double bond of the hydrazone function;
d) the conjugated hydrazone compounds comprising a heteroaromatic group bonded to the nitrogen atom of the hydrazone function and another heteroaromatic group bonded to the carbon atom carrying the double bond of the hydrazone function or comprises a diazoaromatic group bonded to the nitrogen atom of the hydrazone function and a heteroaromatic group including the carbon atom carrying the double bond of the hydrazone function.

2. Colorimetric detection system according to claim 1, wherein, when the conjugated hydrazone compound is a compound of category a), the cyclic group(s) bonded to the carbon atom carrying the double bond of the hydrazone function are carbon-containing cyclic groups and the aromatic group carrying at least one electro-attractive group is an aromatic group carrying at least one NO₂ group.

3. Colorimetric detection system according to either of the preceding claims, wherein the conjugated hydrazone compound meets one of the following formulae (II), (III), (IV), (V), (VI) and (VI'): 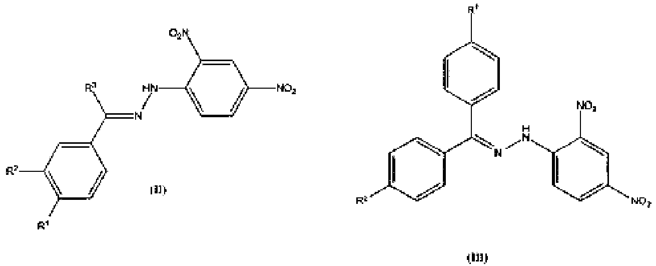 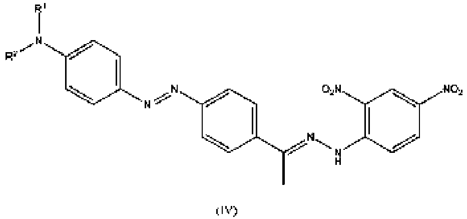 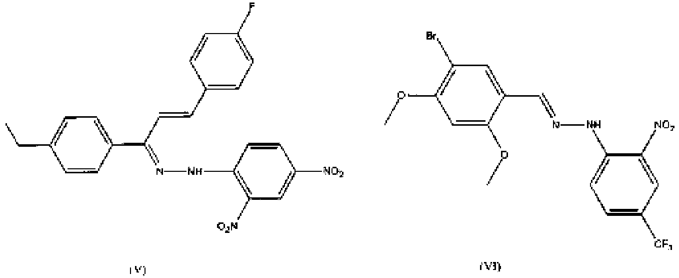 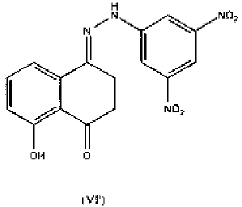 where:
- for the formula (II), R¹ represents -N(CH₃)₂, -CH₃, -OCH₃, -OH, -SCH₃ or - NO₂; R² represents -H or -OCH₃ and R³ represents -H or -CH₃;
- for the formula (III), R¹ and R² represent, independently of one another, - H, -N(CH₃)₂, -OCH₃ or -NO₂;
- for the formula (IV), R¹ and R² represent, independently of one another, - CH₃ or -CH₂ -CH₃.

4. Colorimetric detection system according to claim 3, wherein, when the conjugated hydrazone compound is a compound of formula (II), it is:
- the compound for which R¹ represents -N(CH₃)₂, R² represents -H and R³ represents H;
- the compound for which R¹ represents -CH₃, R² represents H and R³ represents H;
- the compound for which R¹ represents -OCH₃, R² represents -H and R³ represents H;
- the compound for which R¹ represents -OH, R² represents -OCH₃ and R³ represents H;
- the compound for which R¹ represents -SCH₃, R² represents -H, and R³ represents H; or
- the compound for which R¹ represents -NO₂, R² represents -H and R³ represents -CH₃.

5. Colorimetric detection system according to claim 3, wherein, when the conjugated hydrazone compound is a compound of formula (III), it is:
- the compound for which R¹ represents -H and R² represents -H;
- the compound for which R¹ represents -N(CH₃)₂ and R² represents -N(CH₃)₂; or
- the compound for which R¹ represents -OCH₃ and R² represents OCH₃.

6. Colorimetric detection system according to claim 3, wherein, when the conjugated hydrazone compound is a compound of formula (IV), it is:
- the compound for which R¹ represents -CH₃ and R² represents -CH₃; or
- the compound for which R¹ represents -CH₂-CH₃ and R² represents -CH₂-CH₃.

7. Colorimetric detection system according to claim 1, wherein, when the conjugated hydrazone compound is a compound of category a), the cyclic group(s) bonded to the carbon atom carrying the double bond of the hydrazone function are heteroaromatic groups and the aromatic group carrying at least one electro-attractive group is an aromatic group carrying at least one NO₂ group.

8. Colorimetric detection system according to claim 7, wherein the conjugated hydrazone compound meets one of the following formulae (VII), (VIII), (IX), (X) and (XI): 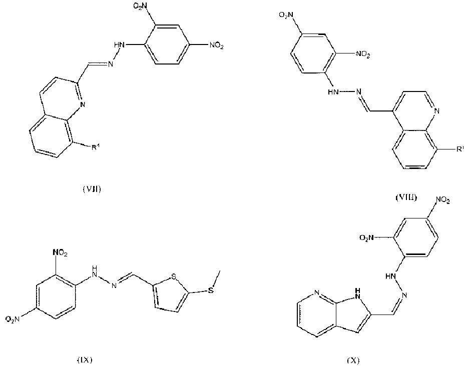 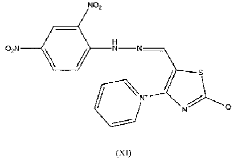 where:
- for formula (VII), R¹ represents -H or -OH;
- for formula (VIII), R¹ represents -H or -OH.

9. Colorimetric detection system according to claim 1, wherein, when the conjugated hydrazone compound is a compound of category b), it meets one of the following formulae (XII) or (XIII):

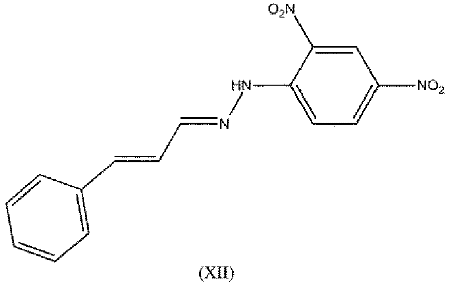

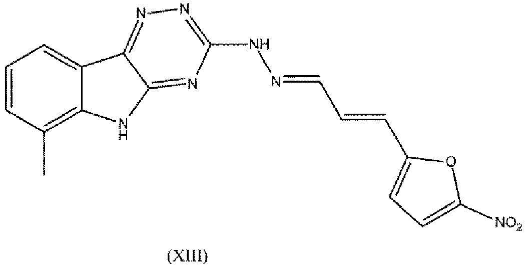

10. Colorimetric detection system according to claim 1, wherein, when the conjugated hydrazone compound is a compound of category c), it meets one of the following formulae (XIV), (XV), (XVI), (XVII), (XVIII), (XIX) and (XX):

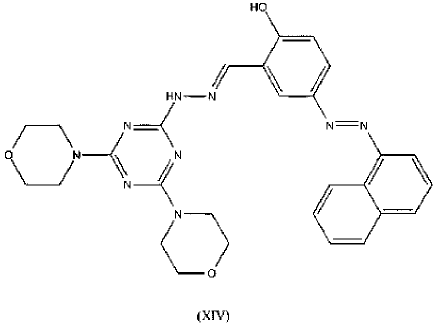

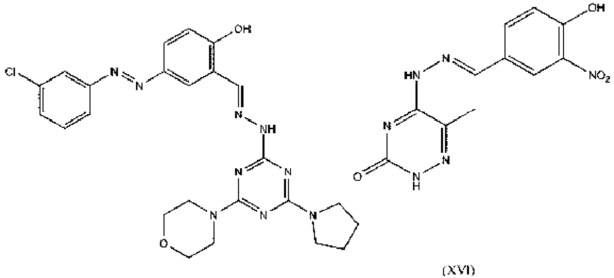

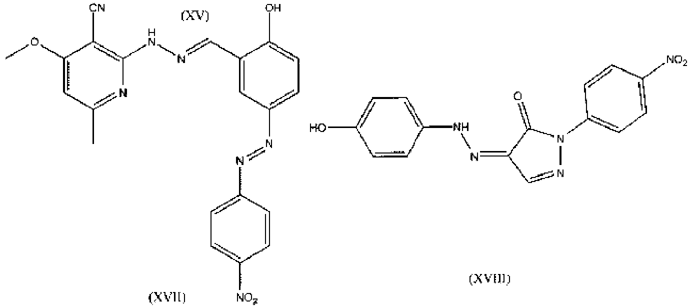

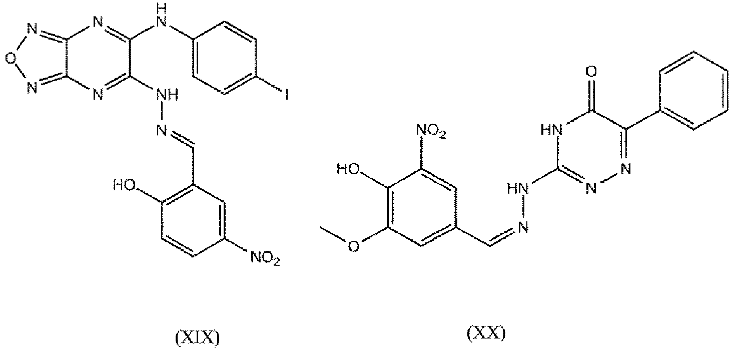

11. Colorimetric detection system according to claim 1, wherein, when the conjugated hydrazone compound is a compound of category d), it meets one of the following formulae (XXI), (XXII), (XXIII) and (XXIV):

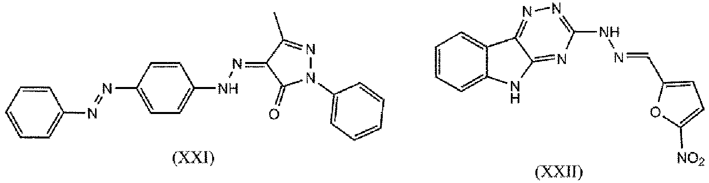

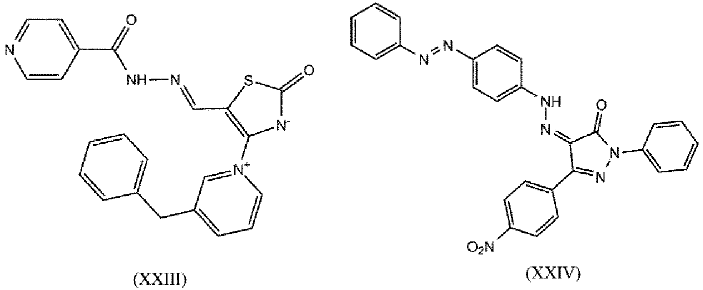

12. Colorimetric detection system according to any one of the preceding claims, wherein the carrier comprises glass fibres or is a paper carrier, preferably comprising glass fibres.

13. Hydrazone compound meeting one of the following formulae (5a), (5b) and (X):

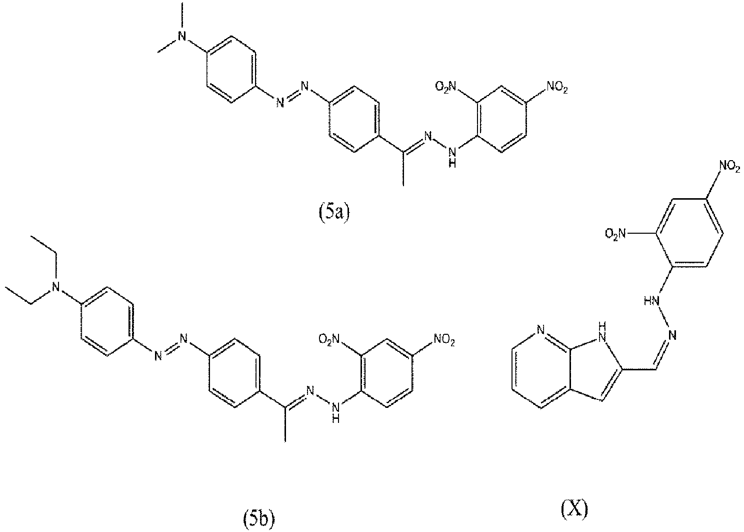

14. Method for detecting the presence or absence of a chemical compound comprising the following steps:
- a step of placing in contact the medium/media in which it is desired to detect the presence or absence of said chemical compound(s) with the detection system as defined according to any one of claims 1 to 12;
- a step of deducing, according to any chromatic curve(s) observed, the presence or absence of said chemical compound.

15. Method according to claim 14, which is a method for detecting the presence or absence of a chemical compound selected from toxic warfare compounds, toxic industrial compounds, pesticides.

16. Method according to claim 15, which is a method for detecting the presence or absence of a chemical compound meeting one of the following formulae (XXV) and (XXVI): 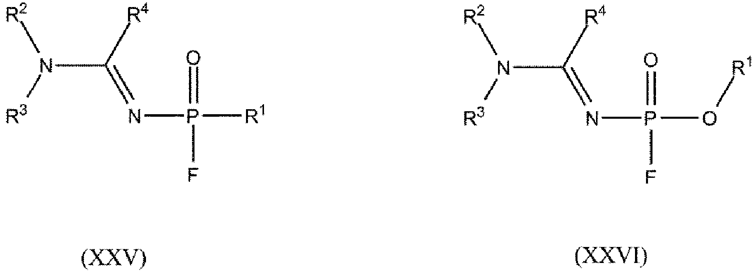 where:
- R¹ represents H or an alkyl group, optionally cyclic, which may comprise up to 10 carbon atoms;
- R² and R³ represent, independently of each other, an alkyl group, optionally cyclic, which may comprise up to 10 carbon atoms;
- R⁴ represents H, an alkyl group, optionally cyclic, which may comprise up to 10 carbon atoms or an amino group.

17. Colorimetric detection kit usable for the detection of a chemical compound comprising the following elements:
- a detection system as defined according to any one of claims 1 to 12; and
- a colorimetric scale making it possible to make a connection between the observed chromatic curve and the chemical compound detected.
